# EUROPEAN PATENT SPECIFICATION

(11) **EP 1 202 628 B1**
(45) Date of publication and mention of the grant of the patent: **13.10.2004**
(21) Application number: 00953925.5
(22) Date of filing: 09.08.2000
(51) Int. Cl.: A01N 43/54, A61K 31/505, A61K 31/52, C07D 487/04

(54) **NOVEL HYPOXANTHINE AND THIOHYPOXANTHINE COMPOUNDS**
HYPOXANTHIN- UND THIOHYPXANTHIN-VERBINDUNGEN
NOUVEAUX COMPOSES D'HYPOXANTHINE ET DE THIOHYPOXANTHINE

(30) Priority: 12.08.1999 US 148623 P
(43) Date of publication of application: 08.05.2002
(73) Proprietor: EURO-CELTIQUE S.A., 2330 Luxembourg (LU)
(72) Inventor: CHASIN, Mark, Manalapan, NJ 07726 (US); HOFER, Peter, CH-4410 Liestal (CH); CAVALLA, David, Cambridge CB1 2DX (GB)
(74) Representative: Maiwald, Walter, Dr. Dipl.-Chem.
(86) International application number: PCT/US2000/021836
(87) International publication number: WO 2001/011967

(56) References cited:
- WO-A-95/00516
- WO-A-96/18399
- WO-A-99/29694
- GB-A- 1 073 039
- US-A- 5 864 037
- DATABASE CAPLUS [Online] HADASSAH MED. SCH., HEB. UNIV., (JERUSALEM, ISRAEL) BERGMANN F. ET AL.: 'Methylation of 6-(methylthio)-8-phenylpurines', XP002935068 Retrieved from STN Database accession no. 1971:435951 & J. CHEM. SOC. vol. 10, 1971, pages 1822 - 1829
- DATABASE CAPLUS [Online] HADASSAH MED. SCH., HEBREW UNIV., (JERUSALEM, ISRAEL) BERGMANN F. ET AL.: 'Influence of 8-substituents on the oxidation of hypoxanthine and 6-thioxopurine by bovine milk xanthine oxidase', XP002935069 Retrieved from STN Database accession no. 1977:67403 & BIOCHIM. BIOPHYS. ACTA vol. 480, no. 1, 1977, pages 39 - 46

## Description

### BACKGROUND OF THE INVENTION

Asthma is a complex disease involving the concerted actions of multiple inflammatory and immune cells, spasmogens, inflammatory mediators, cytokines and growth factors. In recent practice there have been four major classes of compounds used in the treatment of asthma, namely bronchodilators (e.g., β-adrenoceptor agonists), anti-inflammatory agents (e.g., corticosteroids), prophylactic anti-allergic agents (e.g., cromolyn sodium) and xanthines (e.g., theophylline) which appear to possess both bronchodilating and anti-inflammatory activity.

Theophylline has been a preferred drug of first choice in the treatment of asthma. Although it has been touted for its direct bronchodilatory action, theophylline's therapeutic value is now believed to also stem from anti-inflammatory activity. Its mechanism of action remains unclear. However, it is believed that several of its cellular activities are important in its activity as an anti-asthmatic, including cyclic nucleotide phosphodiesterase inhibition, adenosine receptor antagonism, stimulation of catecholamine release, and its ability to increase the number and activity of suppressor T-lymphocytes. While all of these may actually contribute to its activity, only PDE inhibition may account for both the anti-inflammatory and bronchodilatory components. However, theophylline is known to have a narrow therapeutic index and a wide range of untoward side effects which are considered problematic.

Of the activities mentioned above, theophylline's activity in inhibiting cyclic nucleotide phosphodiesterase has received considerable attention recently. Cyclic nucleotide phosphodiesterases (PDEs) have received considerable attention as molecular targets for anti-asthmatic agents. Cyclic 3',5'-adenosine monophosphate (cAMP) and cyclic 3',5'-guanosine monophosphate (cGMP) are known second messengers that mediate the functional responses of cells to a multitude of hormones, neurotransmitters and autocoids. At least two therapeutically important effects could result from phosphodiesterase inhibition, and the consequent rise in intracellular adenosine 3',5'-monophosphate (cAMP) or guanosine 3',5'-monophosphate (cGMP) in key cells in the pathophysiology of asthma. These are smooth muscle relaxation (resulting in bronchodilation) and anti-inflammatory activity.

It has become known that there are multiple, distinct PDE isoenzymes which differ in their cellular distribution. A variety of inhibitors possessing a marked degree of selectivity for one isoenzyme or the other have been synthesized.

The structure-activity relationships (SAR) of isozyme-selective inhibitors has been discussed in detail, e.g., in the article of Theodore J. Torphy, et al., "Novel Phosphodiesterase Inhibitors For The Therapy Of Asthma", Drug News & Prospectives, 6(4) May 1993, pages 203-214. The PDE enzymes can be grouped into five families according to their specificity toward hydrolysis of cAMP or cGMP, their sensitivity to regulation by calcium, calmodulin or cGMP, and their selective inhibition by various compounds. PDE I is stimulated by Ca²⁺/calmodulin. PDE U is cGMP-stimulated, and is found in the heart and adrenals. PDE III is cGMP-inhibited, and inhibition of this enzyme creates positive inotropic activity. PDE IV is cAMP specific, and its inhibition causes airway relaxation, antiinflammatory and antidepressant activity. PDE V appears to be important in regulating cGMP content in vascular smooth muscle, and therefore PDE V inhibitors may have cardiovascular activity.

While there are compounds derived from numerous structure activity relationship studies which provide PDE III inhibition, the number of structural classes of PDE IV inhibitors is relatively limited. Analogues of rolipram, which has the following structural formula (A): 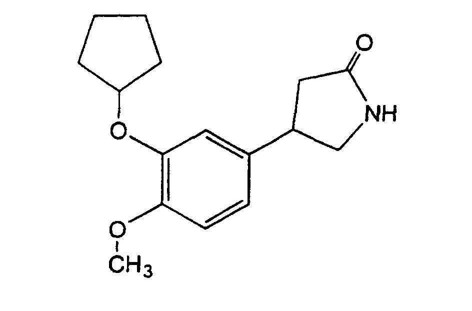 and of RO-20-1724, which has the following structural formula (B): 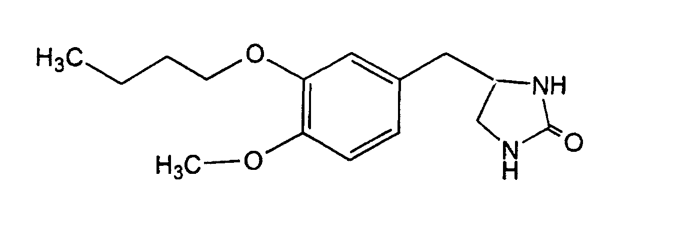 have been studied.

U.S. Patent No. 4,308,278 discloses compounds of the formula (C) 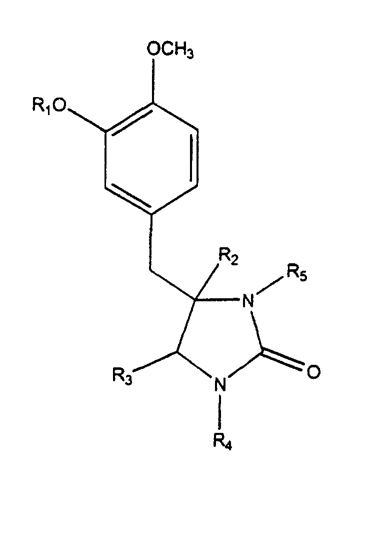 wherein R₁ is (C₃-C₆) cycloalkyl or benzyl; each of R₂ and R₃ is hydrogen or (C₁-C₄) alkyl; R₄ is R₂ or alkoxycarbonyl; and R₅ is hydrogen or alkoxycarbonyl.

Compounds of Formula (D) are disclosed in U.S. Patent No. 3,636,039. These compounds are benzylimidazolidinones which act as hypertensive agents. 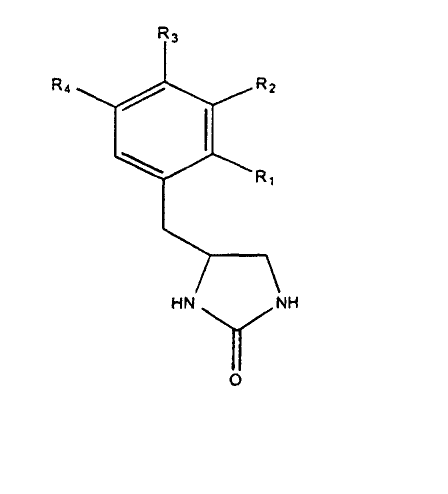 Substituents R₁-R₄ in Formula D represent a variety of groups, including hydrogen and lower alkyl.

PCT publication WO 87/06576 discloses antidepressants of Formula E: 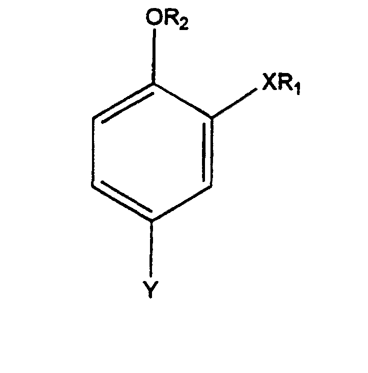 wherein R₁ is a polycycloalkyl group having from 7 to 11 carbon atoms; R₂ is methyl or ethyl; X is O or NH; and Y comprises a mono-or bicyclic heterocyclic group with optional substituents.

Rolipram, which was initially studied because of its activity as an anti-depressant, has been shown to selectively inhibit the PDE IV enzyme and this compound has since become a standard agent in the classification of PDE enzyme subtypes. There appears to be considerable therapeutic potential for PDE IV inhibitors. Early work focused on depression as a CNS therapeutic endpoint and on inflammation, and has subsequently been extended to include related diseases such as dementia and asthma. In-vitro, rolipram, RO20-1724 and other PDE IV inhibitors have been shown to inhibit (1) mediator synthesis/release in mast cells, basophils, monocytes and eosinophils; (2) respiratory burst, chemotaxis and degranulation in neutrophils and eosinophils; and (3) mitogen-dependent growth and differentiation in lymphocytes (The PDE IV Family Of Calcium-Phosphodiesterases Enzymes, John A. Lowe, III, et al., Drugs of the Future 1992, 17(9):799-807).

PDE IV is present in all the major inflammatory cells in asthma including eosinophils, neutrophils, T-lymphocytes, macrophages and endothelial cells. Its inhibition causes down regulation of inflammatory cell activation and relaxes smooth muscle cells in the trachea and bronchus. On the other hand, inhibition of PDE III, which is present in myocardium, causes an increase in both the force and rate of cardiac contractility. These are undesirable side effects for an anti-inflammatory agent. Theophylline, a non-selective PDE inhibitor, inhibits both PDE III and PDE IV, resulting in both desirable anti-asthmatic effects and undesirable cardiovascular stimulation. With this well-known distinction between PDE isozymes, the opportunity for concomitant anti-inflammation and bronchodilation without many of the side effects associated with theophylline therapy is apparent. The increased incidence of morbidity and mortality due to asthma in many Western countries over the last decade has focused the clinical emphasis on the inflammatory nature of this disease and the benefit of inhaled steroids. Development of an agent that possesses both bronchodilatory and antiinflammatory properties would be most advantageous.

It appears that selective PDE IV inhibitors should be more effective with fewer side effects than theophylline. Clinical support has been shown for this hypothesis. Furthermore, it would be desirable to provide PDE IV inhibitors which are more potent and selective than rolipram and therefore have a lower IC₅₀ so as to reduce the amount of the agent required to effect PDE IV inhibition.

In recent years, several different compounds have been suggested as possible therapeutic compositions which achieve the desired PDE IV inhibition without the side effects alluded to above. However, these efforts have been chiefly directed to developing non-specific derivatives of particular classes of compounds, i.e. rolipram analogs, benzoxazoles, adenines, thioxanthines, etc. These efforts, however, have resulted in a myriad of compounds having a wide range of PDE IV IC₅₀'s. Often, the general formulas disclosed yield several compounds which have poor levels of PDE IV inhibition and/or lack sufficient specificity. Consequently, these efforts often provide no assurance that any particular derivative within the formula will have the desired combination of high PDE IV inhibition and selectivity.

WO 95/00516 discloses compounds having PDE-IV inhibitory activity which are synthesized using thiohypoxanthine and hypoxanthine intermediates. The reference does not disclose any utility for these compounds other than their use as intermediates.

WO 96/18399 discloses compounds having PDE-IV inhibitory activity which are xanthines and thioxanthines and derivatives thereof.

### OBJECTS AND SUMMARY OF THE INVENTION

It is accordingly a primary object of the present invention to provide pharmaceutical compositions which are effective selective PDE IV inhibitors.

It is another object of the present invention to provide pharmaceutical compositions which act as effective PDE IV inhibitors with lower PDE III inhibition.

It is another object of the present invention to provide methods of preparing a pharmaceutical composition for treating a patient requiring PDE IV inhibition.

It is another object of the present invention to provide pharmaceutical compositions for treating disease states associated with abnormally high physiological levels of inflammatory cytokines, including tumor necrosis factor.

It is another object of the present invention to provide a method of synthesizing the new compounds of this invention.

It is another object of the present invention to provide a method of preparing a pharmaceutical composition for treating a patient suffering from disease states such as asthma; allergies; inflammation; depression; dementia, including Alzheimer's disease, vascular dementia, and multi-in-farct dementia; a disease caused by Human Immunodeficiency Virus; and disease states associated with abnormally high physiological levels of inflammatory cytokines.

Other objects and advantages of the present invention will become apparent from the following detailed description thereof.

With this and other objects in view, the present invention is directed to pharmaceutical compositions comprising a pharmaceutical acceptable carrier and a compound having the general formula (I): 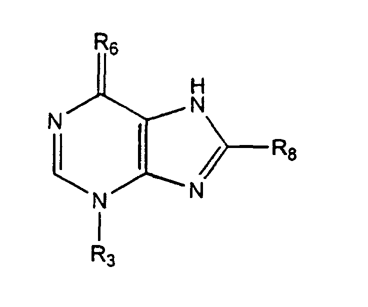 wherein:
R₆ = S or O
R₃ is selected from the group consisting of C₁ - C₈ linear or branched alkyl; C₂ - C₈ linear or branched alkene; C₂ - C₈ linear or branched alkyne; C₃₋₈ cycloalkyl; Q; and K;
R₈ is selected from the group consisting of H, C₁ - C₈ linear or branched alkyl; C₂ - C₈ linear or branched alkene; C₂ - C₈ linear or branched alkyne; C₃₋₈ cycloalkyl; Q; and K; wherein
   Q has the general formula: 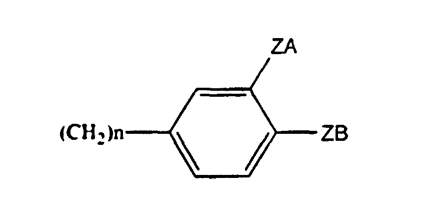 wherein;
      n = 0 or 1;
      Z = a bond, CH₂, NH, O or S;
      A and B can form a ring by adding 1-3 CH₂ groups when Z = CH₂, NH, O or S; and
      A and B are not in a ring when Z = a bond, wherein A and B are independently selected from the group consisting of hydrogen; halogen; C₁ - C₈ alkyl; C₁ - C₈ alkoxy; C₃ - C₈ cycloalkyl; C₃ - C₈ cycloalkoxy; hydroxy; phenyl; benzyl; and benzyloxy; wherein said phenyl, benzyl and benzyloxy are optionally substituted with halogen, C₁ - C₈ alkyl, C₁ - C₈ alkoxy, C₃ - C₈ cycloalkyl, C₃ - C₈ cycloalkoxy and hydroxy;
K has the general formula: 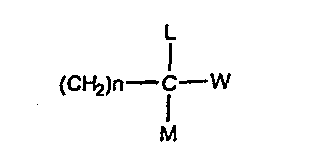 wherein;
   n = 0 or 1;
   L and M are independently selected from the group consisting of hydrogen and methyl;
   W is selected from the group consisting of Q; hydroxy; benzyloxy optionally substituted with halogen, C₁ - C₈ alkyl, C₁ *-* C₈ alkoxy, C₃ *-* C₈ cycloalkyl, C₃ - C₈ cycloalkoxy and hydroxy; aryl; heteroaryl; and a heterocyclic ring;
   provided that when R₃ is methyl, R₈ is not hydrogen;

The present invention is also directed to methods of preparing a pharmaceutical composition for treating a patient suffering from disease states such as asthma; allergies; inflammation; depression; dementia, including Alzheimer's disease, vascular dementia, and multi-in-farct dementia; a disease caused by Human Immunodeficiency Virus; and disease states associated with abnormally high physiological levels of inflammatory cytokines, comprising preparation of a pharmaceutical composition with a compound having the general formula (I): 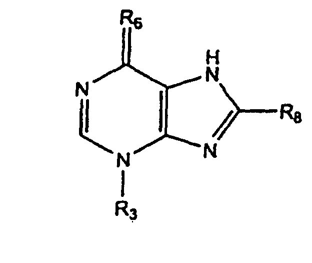 wherein;
R₆ = S or O
R₃ is selected from the group consisting of C₁ - C₈ linear or branched alkyl; C₂ - C₈ linear or branched alkene; C₂ - C₈ linear or branched alkyne; C₃₋₈ cycloalkyl; Q; and K;
R₈ is selected from the group consisting of H, C₁ - C₈ linear or branched alkyl; C₂ - C₈ linear or branched alkene; C₂ - C₈ linear or branched alkyne; C₃₋₈ cycloalkyl; Q; and K; wherein
   Q has the general formula: 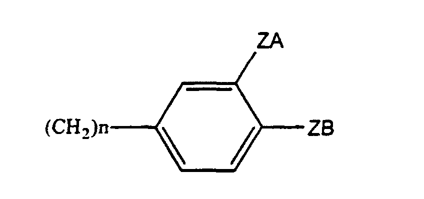 wherein;
      n = 0 or 1;
      Z = a bond, CH₂, NH, O or S;
      A and B can form a ring by adding 1-3 CH₂ groups when Z = CH₂, NH, O or S; and
      A and B are not in a ring when Z = a bond, wherein A and B are independently selected from the group consisting of hydrogen; halogen; C₁ - C₈ alkyl; C₁ - C₈ alkoxy; C₃ - C₈ cycloalkyl; C₃ - C₈ cycloalkoxy; hydroxy; phenyl; benzyl; and benzyloxy; wherein said phenyl, benzyl and benzyloxy are optionally substituted with halogen, C₁ - C₈ alkyl, C₁ - C₈ alkoxy, C₃ - C₈ cycloalkyl, C₃ - C₈ cycloalkoxy and hydroxy;
   K has the general formula: 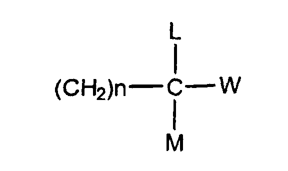 wherein;
      n=0 or 1;
      L and M are independently selected from the group consisting of hydrogen and methyl;
      W is selected from the group consisting of Q; hydroxy; benzyloxy optionally substituted with halogen, C₁ - C₈ alkyl, C₁ - C₈ alkoxy, C₃ - C₈ cycloalkyl, C₃ - C₈ cycloalkoxy and hydroxy; aryl; heteroaryl; and a heterocyclic ring;
      provided that when R₃ is methyl, R₈ is not hydrogen.

In particular embodiments of the invention, R³ is benzyl substituted in two positions with an alkoxy e.g. methoxy, or a cycloalkoxy e.g. cyclopentyl, wherein said alkyl moiety of said alkoxy or cycloalkoxy substituent is optionally substituted in one position with hydroxy. In particularly preferred embodiments, R₃ is cyclopentyloxy-methoxy-benzyl (e.g. 3-cyclopentyloxy-4-methoxy-benzyl).

In other embodiments of the invention, R₃ is hydrogen, methyl, ethyl, propyl, butyl, pentyl, hexyl, benzyl, methyl-butyl (e.g. 2-methyl-butyl or 4-methyl-butyl), benzyloxy-methoxy-benzyl (e.g. 3-benzyloxy-4-methoxy-benzyl), dimethoxy-benzyl (e.g. 3-4-dimethoxy-benzyl), hydroxy-methoxy-benzyl (e.g. 3-hydroxy-4-methoxy-benzyl), methyl-benzdioxole (e.g. 1,3-benzodioxole-5-methyl), chloro-benzyl (e.g. 3-chloro-benzyl or 4-chloro-benzyl), methoxy-benzyl (e.g. 4-methoxy-benzyl), dimethoxy-benzyl (e.g. 3-4 dimethoxy-benzyl), or other groups which fall into the general description of R₃ as set forth above.

In other embodiments of the invention, R₈ is hydrogen, methyl, ethyl, propyl, isopropyl (1-methyl-ethyl), cyclopropyl, hydroxy-methyl-ethyl (e.g. 1-hydroxy-1-methyl-ethyl), isopropene (1-methyl-ethylene), benzyloxy-propyl (e.g. 1-benzyloxy-1-methyl-ethyl), benzyloxymethyl, methoxybenzyloxy-propyl (e.g. 1-(4-methoxybenzyloxy)-1-methyl-ethyl), halobenzyloxy-propyl (e.g. 1-(4-fluorobenzyloxy)-1-methyl-ethyl), or other groups which fall into the general description of R₈ as set forth above.

In certain embodiments of the invention, at least one of R₃ and R₈ is not hydrogen

### DETAILED DESCRIPTION OF THE INVENTION

As used herein, the following terms are intended to have the meaning as understood by persons of ordinary skill in the art, and are specifically intended to include the meanings set forth below:

As used herein, the term "alkyl" means a linear or branched saturated aliphatic hydrocarbon group having a single radical. Examples of alkyl groups include methyl, propyl, isopropyl, butyl, n-butyl, isobutyl, sec-butyl, tert-butyl, and pentyl. A branched alkyl means that one or more alkyl groups such as methyl, ethyl or propyl, replace one or both hydrogens in a -CH₂- group of a linear alkyl chain.

The term "cycloalkyl" means a non-aromatic monocyclic hydrocarbon ring system having a single radical. Exemplary monocyclic cycloalkyl rings include cyclopropyl, cyclopentyl, and cyclohexyl.

The term "alkoxy" means an alkyl-O-group in which the alkyl group is as previously defined, to include a linear or branched saturated aliphatic hydrocarbon group having a single radical. Exemplary alkoxy groups include methoxy, ethoxy, n-propoxy, i-propoxy, and n-butoxy.

The term "cycloalkoxy" means a cycloalkyl-O-group in which the cycloalkyl group is as previously defined, to include non-aromatic mono- or multicyclic hydrocarbon ring systems having a single radical. Exemplary cycloalkoxy groups include cyclopentyloxy.

The term "aryl" means a carbocyclic aromatic ring system containing one, two or three rings which may be attached together in a pendent manner or fused, and containing a single radical. Exemplary aryl groups include phenyl and naphthyl.

The term "heterocyclic" or "heterocyclyl" means cyclic compounds having one or more heteroatoms (atoms other than carbon) in the ring, and having a single radical. The ring may be saturated, partially saturated and unsaturated, and the heteroatoms may be selected from the group consisting of nitrogen, sulfur and oxygen. Examples of saturated heterocyclic radicals include saturated 3 to 6- membered heteromonocyclic groups containing 1 to 4 nitrogen atoms, such as pyrrolidinyl, imidazolidinyl, piperidino, piperazinyl; saturated 3- to 6-membered heteromonocyclic groups containing 1 to 2 oxygen atoms and 1 to 3 nitrogen atoms, such as morpholinyl; saturated 3- to 6- membered heteromonocyclic groups containing 1 to 2 sulfur atoms and 1 to 3 nitrogen atoms, such as thiazolidinyl. Examples of partially saturated heterocyclic radicals include dihydrothiophene, dihydropyran, and dihydrofuran.

The term "heteroaryl" means unsaturated heterocyclic radicals, wherein heterocyclic is as previously described. Exemplary heteroaryl groups include unsaturated 3 to 6 membered heteromonocyclic groups containing 1 to 4 nitrogen atoms, such as pyrrolyl, pyridyl, pyrimidyl, and pyrazinyl; unsaturated condensed heterocyclic groups containing 1 to 5 nitrogen atoms, such as indolyl, quinolyl, isoquinolyl; unsaturated 3 to 6 membered heteromonocyclic groups containing an oxygen atom, such as furyl; unsaturated 3 to 6 membered heteromonocyclic groups containing a sulfur atom, such as thienyl; unsaturated 3 to 6 membered heteromonocyclic groups containing an oxygen atom and 1 to 3 nitrogen atoms, such as oxazolyl; unsaturated condensed heterocyclic groups containing an oxygen atom and 1 to 3 nitrogen atoms, such as benzoxazolyl; unsaturated 3 to 6 membered heteromonocyclic groups containing a sulfur atom and 1 to 3 nitrogen atoms, such as thiazolyl; unsaturated condensed heterocyclic group containing a sulfur atom and 1 to 3 nitrogen atoms, such as benzothiazolyl. The term "heteroaryl" also includes unsaturated heterocyclic radicals, wherein heterocyclic is as previously described, in which the heterocyclic group is fused with an aryl group, in which aryl is as previously described. Exemplary fused radicals include benzofuran, benzdioxole and benzothiophene.

As used herein, the term "patient" includes both human and other mammals.

The present invention also includes organic and inorganic salts, hydrates, esters, prodrugs and metabolites of the compounds of formula I.

The compounds of the present invention can be administered to anyone requiring PDE IV inhibition. Administration may be orally, topically, by suppository, inhalation or insufflation, or parenterally.

The present invention also encompasses all pharmaceutically acceptable salts of the foregoing compounds. One skilled in the art will recognize that acid addition salts of the presently claimed compounds may be prepared by reaction of the compounds with the appropriate acid via a variety of known methods.

Various oral dosage forms can be used, including such solid forms as tablets, gelcaps, capsules, caplets, granules, lozenges and bulk powders and liquid forms such as emulsions, solution and suspensions. The compounds of the present invention can be administered alone or can be combined with various pharmaceutically acceptable carriers and excipients known to those skilled in the art, including but not limited to diluents, suspending agents, solubilizers, binders, disintegrants, preservatives, coloring agents, lubricants and the like.

When the compounds of the present invention are incorporated into oral tablets, such tablets can be compressed, tablet triturates, enteric-coated, sugar-coated, film-coated, multiple compressed or multiple layered. Liquid oral dosage forms include aqueous and nonaqueous solutions, emulsions, suspensions, and solutions and/or suspensions reconstituted from non-effervescent granules, containing suitable solvents, preservatives, emulsifying agents, suspending agents, diluents, sweeteners, coloring agents, and flavoring agents. When the compounds of the present invention are to be injected parenterally, they may be, e.g., in the form of an isotonic sterile solution. Alternatively, when the compounds of the present invention are to be inhaled, they may be formulated into a dry aerosol or may be formulated into an aqueous or partially aqueous solution.

In addition. when the compounds of the present invention are incorporated into oral dosage forms, it is contemplated that such dosage forms may provide an immediate release of the compound in the gastrointestinal tract, or alternatively may provide a controlled and/or sustained release through the gastrointestinal tract. A wide variety of controlled and/or sustained release formulations are well known to those skilled in the art, and are contemplated for use in connection with the formulations of the present invention. The controlled and/or sustained release may be provided by, e.g., a coating on the oral dosage form or by incorporating the compound(s) of the invention into a controlled and/or sustained release matrix.

Specific examples of pharmaceutically acceptable carriers and excipients that may be used to formulate oral dosage forms, are described in the Handbook of Pharmaceutical Excipients, American Pharmaceutical Association (1986), incorporated by reference herein. Techniques and compositions for making solid oral dosage forms are described in Pharmaceutical Dosage Forms: Tablets (Lieberman, Lachman and Schwartz, editors) 2nd edition, published by Marcel Dekker, Inc., incorporated by reference herein. Techniques and

compositions for making tablets (compressed and molded), capsules (hard and soft gelatin) and pills are also described in Remington's Pharmaceutical Sciences (Arthur Osol, editor), 1553-1593 (1980), incorporated herein by reference. Techniques and composition for making liquid oral dosage forms are described in Pharmaceutical Dosage Forms: Disperse Systems, (Lieberman, Rieger and Banker, editors) published by Marcel Dekker, Inc., incorporated herein by reference.

When the compounds of the present invention are incorporated for parenteral administration by injection (e.g., continuous infusion or bolus injection), the formulation for parenteral administration may be in the form of suspensions, solutions, emulsions in oily or aqueous vehicles, and such formulations may further comprise pharmaceutically necessary additives such as stabilizing agents, suspending agents, dispersing agents, and the like. The compounds of the invention may also be in the form of a powder for reconstitution as an injectable formulation.

The dose of the compounds of the present invention is dependent upon the affliction to be treated, the severity of the symptoms, the route of administration, the frequency of the dosage interval, the presence of any deleterious side-effects, and the particular compound utilized, among other things.

Compounds of the invention falling into the genus of PDE IV inhibitors of general formula (I), include:
3-methyl-hypoxanthine;
3-butyl-hypoxanthine;
3-butyl-thiohypoxanthine;
3-ethyl-hypoxanthine;
3-ethyl-thiohypoxanthine;
3,8-diethyl-hypoxanthine;
3,8-diethyl-thiohypoxanthine;
3-ethyl-8-cyclopropyl-hypoxanthine;
3-ethyl-8-cyclopropyl-thiohypoxanthine;
3-propyl-hypoxanthine;
3-hexyl-hypoxanthine;
3-hexyl-thiohypoxanthine;
3-benzyl-hypoxanthine;
3-benzyl-thiohypoxanthine;
3-(4-methyl-butyl)-hypoxanthine;
3-(4-methyl-butyl)-thiohypoxanthine;
3-(2-methyl-butyl)-hypoxanthine;
3-(2-methyl-butyl)-thiohypoxanthine;
3-(3-cyclopentyloxy-4-methoxy-benzyl)-hypoxanthine;
3-(3-cyclopentyloxy-4-methoxy-benzyl)-8-(1-hydroxy-1-methyl-ethyl)-hypoxanthine;
3-(3-cyclopentyloxy-4-methoxy-benzyl)-8-(1-methyl-ethylene)-hypoxanthine;
3-(3-cyclopentyloxy-4-methoxy-benzyl)-8-(1-benzyloxy-1-methyl-ethyl)-hypoxanthine;
3-(3-cyclopentyloxy-4-methoxy-benzyl)-8-(benzyloxymethyl)-hypoxanthine;
3-(3-cyclopentyloxy-4-methoxy-benzyl)-8-(1-methyl-ethyl)-hypoxanthine;
3-(3-cyclopentyloxy-4-methoxy-benzyl)-8-(1(4-methoxybenzyloxy)-1-methyl-ethyl)-hypoxanthine;
3-(3-cyclopentyloxy-4-methoxy-benzyl)-8-(1(4-fluorobenzyloxy)-1-methyl-ethyl)-hypoxanthine;
3-(3-benzyloxy-4-methoxy-benzyl)-8-(1-benzyloxy-1-methyl-ethyl)-hypoxanthine;
3-(3-4-dimethoxy-benzyl)-8-(1-benzyloxy-1-methyl-ethyl)-hypoxanthine;
3-(3-benzyloxy-4-methoxy-benzyl)-8-(1-methyl-ethyl)-hypoxanthine;
3-(3-hydroxy-4-methoxy-benzyl)-8-(1-methyl-ethyl)-hypoxanthine;
3-(3-4-dimethoxy-benzyl)-8-(1-methyl-ethyl)-hypoxanthine;
3-(1,3-benzodioxole-5-methyl)-8-(1-methyl-ethyl)-hypoxanthine;
3-(4-chloro-benzyl)-8-(1-methyl-ethyl)-hypoxanthine;
3-(3-chloro-benzyl)-8-(1-methyl-ethyl)-hypoxanthine;
3-(4-methoxy-benzyl)-8-(1-methyl-ethyl)-hypoxanthine;
3-(3-4-dimethoxy-benzyl)-8-(1-(4-fluorobenzyloxy)-1-methyl-ethyl)-hypoxanthine;
and pharmaceutically acceptable salts thereof.

When certain of the above-identified compounds may exist in geometric or stereoisometric forms, the present invention contemplates all such compounds.

### DETAILED DESCRIPTION OF PREFERRED EMBODIMENTS

The following examples illustrate various aspects of the invention.

### Example 1

### 3,8-Diethyl-hypoxanthine

3,8-diethyl-2-thioxanthine (18.9 g) was dissolved in 370 ml of 2N NaOH Nickel aluminum alloy (75.6 g) (1.4M of Al and 0.6M of Ni) was added in portions over 1.5 hrs at 65°C. After a further 0.5 hr at 65-70°C the reaction product was filtered, washed with 200 ml of 1N NaOH and the filtrate neutralized with 183 ml of 5N HCl to pH 7. The formed aluminum hydroxide was filtered off, the filtrate concentrated to dryness, the residue suspended in 500 ml of absolute ethanol at 90°C, and the insoluble NaCl filtered off and washed. The filtrate was concentrated to dryness, dissolved in 200 ml of chloroform, filtered and concentrated to dryness again. The residue was crystallized from 150 ml of ethanol to give 3,8-di-ethyl-hypoxanthine (12.68 g) with mp (sublimation at 220°C) 305-307°C under decomposition.

### Example 2

### 3,8-Diethyl-6-thiohypoxanthine

The product of Example 1 (8.65 g) and phosphorus pentasulfide (12.0 g) was refluxed in 150 ml of pyridine for 1 hr. Under cooling 59.4 ml of 2N NaOH was added dropwise, the solid filtered off and washed with water. The filtrate was concentrated in vacuo to dryness and the residue suspended in 200 ml of water and collected. The filtrate was extracted three times with 600 ml of chloroform. The residue of the organic phase was combined with the solid collected (total 6.08 g), dissolved in 500 ml of chloroform and filtered through 24 g of silicagel. Fractions 2 and 3 eluted 4.63 g of crude product which was crystallized from 120 ml of methanol to give 3,8-diethyl-6-thiohypoxanthine (3.58 g) with mp (sublimation at 210°C) 250-270°C under decomposition. A second crop gave 0.58 g.

| Elemental analysis: | | | |
|---|---|---|---|
| % calc | C 51.90 | H 5.81 N 26.90 | S 15.40 |
| % found | C 51.76 | H 6.01 N 26.82 | S 15.64 |

### Example 3

### 3-(3-benzyloxy-4-methoxy-benzyl)-8-(1-benzyloxy-1-methyl-ethyl)-hypoxanthine

### A. 1-(3-Benzyloxy-4-methoxy-benzyl)-2-thiourea

A solution of 3-hydroxy-4-methoxy-benzyl alcohol (61.67 g, 400 mmole) in 1-propanol (600ml) was treated at 60°C with 97% t-BuOK (55.52 g, 480 mmole), at 90°C with benzyl chloride (66.57 ml, 560 mmole) and the resulting mixture heated under reflux for 2 hours. Then at 90°C a second batch of t-BuOK (9.25 g, 80 mmole) was added. After a further hour at reflux a third batch of t-BuOK (9.25 g, 80 mmol) and a second batch of benzyl chloride (9.51 ml, 80 mmole) was added at 90°C. After a further 2.5 hours at reflux the mixture was cooled to room temperature and the solid filtered off. The solvents were removed *in vacuo*, the residue treated with water (300ml) and one-third of the solvent removed *in vacuo*. Water (100ml) was added to the residue, which was then distilled off *in vacuo* and the procedure repeated. The resulting suspension was filtered, the solid was collected, dried and triturated with petroleum ether (2 x 600ml) to give 3-benzyloxy-4-methoxy-benzyl alcohol, (86.21 g, 88.2%) mp 62-65°C.

Thionyl chloride (64 ml) was added over 10 minutes to a stirred solution of the above alcohol in dichloromethane (500ml). After 20 minutes the mixture was evaporated to dryness, *in vacuo*, toluene (2 x 75 ml) added, and evaporation *in vacuo* repeated to give crude 3-benzyloxy-4-methoxy-benzyl chloride (97.85 g, 105.5%). The chloride (353 mmole) was dissolved in acetone, (400ml) treated with sodium thiocyanate, (57.22 g ,706 mmole) homogenized and heated under reflux for 1.5 hours. The solid was filtered off at room temperature and the solvent evaporated *in vacuo*. The residue was suspended in water (600 ml) to give a solution of pH 2, and neutralized with sodium bicarbonate solution. After crystallization the solid was collected, dried, dissolved in dichloromethane (300ml), dried (Na₂SO₄), treated with charcoal, filtered and evaporated to dryness. The residue was crystallized from petroleum ether (400ml) to give 3-benzyloxy-4-methoxy-benzyl thiocyanate (95.65 g, 95.0%), mp 70-74°C. The thiocyanate (335 mmole) was heated under reflux in n-valeronitrile (280ml) for 2 hours and evaporated to dryness to give 98.4 g of crude product. This was dissolved in THF (200ml) and treated with 32% aqueous ammonia solution (101ml) at room temperature. After 3 hours the thiourea was collected at 10°C and washed with diethyl ether to give 1-(3-benzyloxy-4-methoxy-benzyl)-2-thiourea (63.08 g, 62.2%), mp 179-181°C. The filtrate was evaporated to dryness and the residue crystallized from dichloromethane to give 11.51 g (11.3%) as a second crop.

### B. 6-Amino-1-(3-benzyloxy-4-methoxy-benzyl)-2-thiouracil

1-(3-Benzyloxy-4-methoxy-benzyl)-2-thiourea (72.58 g, 240 mmole) was added to a solution of 97% t-BuOK (30.54 g, 264 mmole) in isopropanol (300 ml), the mixture heated under reflux until dissolution was complete, then ethyl cyanoacetate (26.1.ml, 245 mmole) was added. After 5 hours at reflux, the mixture was cooled slightly, another batch of t-BuOK (2.78 g ,24 mmole) and ethyl cyanoacetate (5.12 ml, 48 mmole) was added ,and the mixture heated under reflux for a further 15 hours. The reaction mixture was cooled to room temperature, poured onto water (1.21) and neutralized with cooling to pH 8 with 5M HCl (43ml). After 1 hour stirring the solid was collected at 10°C and washed first with water (180ml), then saturated sodium bicarbonate solution (60ml), isopropanol (60ml) and finally cold water (500ml). The crude material was suspended in 0.5M NaOH (1.4 l) and isopropanol (350ml). The insoluble part was filtered off and washed with 0.1M NaOH and water to give recovered thiourea (10.45 g, 14.4%). The filtrate was neutralized to pH 8 with 2M phosphoric acid (175 ml), crystallized over night, the solid collected and washed with the above three component liqours and water to give the crude thiouracil (73.33 g). This product was suspended in hot acetone (600 ml), concentrated to 500ml and collected at 0-5°C to give 6-amino-1-(3-benzyloxy-4-methoxy-benzyl)-2-thiouracil (65.73 g, 74.1%), mp 239-240°C.

### C. 1-(3-Benzyloxy-4-methoxy-benzyl)-5,6-diamino-2-thiouracil

6-amino-1-(3-benzyloxy-4-methoxy-benzyl)-2-thiouracil (36.94 g, 100 mmole) was dissolved in DMSO (74ml), diluted with THF (185ml) and treated with 85% phosphoric acid (7.46 ml). At 55-60°C, 4M sodium nitrite (30 ml, 120 mmole) was added slowly. After 30 minutes, methanol (10 ml) was added, the reaction was cooled to 30°C, and a suspension of 85% sodium dithionite (40.96 g, 200 mmole) in water (80ml) was added slowly. After the addition of water (200ml) the solvent was removed *in vacuo* and the suspension diluted with water to 1 l. The solid was collected at 0-5°C to give crude 1-(3-benzyloxy-4-methoxy-benzyl)-5,6-diamino-2-thiouracil (40.58 g).

### D. 6-Amino-1-(3-benzyloxy-4-methoxy-benzyl)-5-(2-benzyloxy-2-methyl-propionylamino)-2-thiouracil

A solution of 2-benzyloxy-2-methyl-propionyl chloride (30.70 g, 144 mmole) in THF (100ml) was added at 0-5°C over 15 minutes to a stirred suspension of 1-(3-benzyloxy-4-methoxy-benzyl)-5,6-diamino-2-thiouracil (40.58 g, 100 mmole) and triethylamine (31.4 ml, 225 mmole) in THF (400ml). After 1 hour the solid was filtered off and the solution evaporated to dryness *in vacuo*. The residue was suspended in a mixture of diethyl ether (400ml), water (100ml) and saturated sodium bicarbonate solution (60ml). After crystallization for 60 hours the solid was collected and washed with ether and water to give 6-amino-1-(3-benzyloxy-4-methoxy-benzyl)-5-(2-benzyloxy-2-methyl-propionylamino)-2-thiouracil (42.23 g, 75.3%). Crystallization from ethyl acetate gave mp 123-125/184-187°C.

### E. 3-(3-Benzyloxy-4-methoxy-benzyl)-8-(1-benzyloxy-1-methyl-ethyl)-2-thioxanthine

6-Amino-1-(3-benzyloxy-4-methoxy-benzyl)-5-(2-benzyloxy-2-methyl-propionylamino)-2-thiouracil (46.54 g, 83 mmole) and 97% t-BuOK (38.41 g, 332 mmole) were heated under reflux in isopropanol (460ml) for 50 minutes. The solvent was removed *in vacuo*, the residue dissolved in water (300ml), treated twice with 5 g of charcoal, filtered, water added to a total volume of 500 ml and pH adjusted to neutral with a mixture of 5M HCl (60ml) and sodium bicarbonate solution. The solid was collected at 10°C to give 41.62 g of crude product, which was dissolved in dichloromethane (60ml) and chromatographed over silicagel (126 g) using dichloromethane as eluant. Crystallization from methanol gave 3-(3-benzyloxy-4-methoxy-benzyl)-8-(1-benzyloxy-1-methyl-ethyl)-2-thioxanthine, (31.5 g, 69.9%) mp 290-302°C(dec).

### F. 3-(3-Benzyloxy-4-methoxy-benzyl)-8-(1-benzyloxy-1-methyl-ethyl)-hypoxanthine

3-(3-Benzyloxy-4-methoxy-benzyl)-8-(1-benzyloxy-1-methyl-ethyl)-2-thioxanthine (26.05 g, 48 mmole) was heated under reflux in 1-propanol (700ml) with Raney-nickel (29g) (treated with 0.1% aqueous acetic acid) for 1.5 hours. The nickel was filtered off and the solvent evaporated *in vacuo*. The residue was dissolved in dichloromethane (300ml), extracted with sodium carbonate solution and evaporated again to dryness. The residue was dissolved in methanol (150ml), treated with charcoal, filtered, concentrated and crystallized to give 3-(3-benzyloxy-4-methoxy-benzyl)-8-( 1-benzyloxy-1-methyl-ethyl)-hypoxanthine (15.81 g, 64.5%), mp 78-86°C (containing methanol). A second crop gave 2.29 g (9.3%) of hypoxanthine.

### Example 4

### 3-(3-benzyloxy-4-methoxy-benzyl)-8-isopropyl-hypoxanthine

### A. 6-Amino-1-(3-benzyloxy-4-methoxy-benzyl)-5-isobutyrylamino-2-thiouracil

4M sodium nitrite solution (46.6 ml, 186 mmole) was added within 5 minutes at 55°C with stirring to a solution of 6-amino-1-(3-benzyloxy-4-methoxy-benzyl)-2-thiouracil (53.00 g, 144 mmole) and 85% phosphoric acid (12.09 ml, 179 mmole) in DMF (550 ml). After 1.5 hours the reaction mixture was cooled to 35°C and treated with a suspension of 85% sodium dithionite (58.77 g, 287 mmole) in water (138 ml). After 30 minutes isobutyric anhydride (72 ml, 215 mmole) was added. After 1 hour the suspension was diluted slowly with 1M NaOH (790 ml) and water (1950ml) (pH changed from 3 to 7). After stirring over night sodium bicarbonate (24.2 g) was added to give a pH of 7.5. The solid was collected, washed with 0.2M sodium bicarbonate solution followed by water, then dried to give crude thiouracil (56.10 g, 86.0%). Trituration with acetone gave 6-amino-1-(3-benzyloxy-4-methoxy-benzyl)-5-isobutyrylamino-2-thiouracil, mp 240-244°C.

### B. 3-(3-Benzyloxy-4-methoxy-benzyl)-8-isopropyl-2-thioxanthine

6-Amino-1-(3-benzyloxy-4-methoxy-benzyl)-5-isobutyrylamino-2-thiouracil (56.10 g, 123 mmole) was heated under reflux in 1M NaOH (560 ml) solution for 40 minutes. After cooling to room temperature the solid was removed by filtration, the solution treated twice with charcoal (2.7g), filtered and neutralized with 5M HCl (95 ml) to pH 8. The solid was collected, washed with 0.1M sodium bicarbonate solution and water then dried to give crude product (38.65 g, 71.7%). Crystallization from THF and methanol gave still impure product (34.65 g), which was dissolved in 1M NaOH (340ml), treated twice with charcoal (3.4 g), filtered, diluted with methanol (80ml) and neutralized with 85% phosphoric acid (13ml). The solid was collected and washed with water (1.5l) until a neutral pH to give 3-(3-benzyloxy-4-methoxy-benzyl)-8-isopropyl-2-thioxanthine (28.99 g, 53.8%), mp 280-281°C.

### C. 3-(3-benzyloxy-4-methoxy-benzyl)-8-isopropyl-hypoxanthine

3-(3-Benzyloxy-4-methoxy-benzyl)-8-isopropyl-2-thioxanthine (21.83 g, 50 mmole) was heated under reflux in 1-propanol (700ml) with Raney-nickel (30g) (pre-treated with 0.1% of aqueous acetic acid). After 4 hours the nickel was filtered off and washed with hot propanol and chloroform. The solution was evaporated to dryness *in vacuo*. The residue was dissolved in chloroform, extracted with 1M sodium carbonate solution, dried (Na₂SO₄),treated twice with charcoal (0.8g), filtered and evaporated to dryness *in vacuo*. The residue was suspended in hot acetone (250ml), concentrated and the solid collected at 0-5°C to give 3-(3-benzyloxy-4-methoxy-benzyl)-8-isopropyl-hypoxanthine (15.27 g, 75.5%), mp 233-235°C.

### Example 5

### 8-(1-benzyloxy-1-methyl-ethyl)-3-(3-cyclopentyloxy-4-methoxy-benzyl)-hypoxanthine

### A. 6-Amino-5-(2-benzyloxy-2-methyl-propionylamino)-1-(3-cyclopentyloxy-4-methoxybenzyl)-2-thiouracil

A solution with 26.39 g (124 mM) of 2-benzyloxy-2-methyl-propionyl chloride in 86 ml of THF was added at 5-10°C within 20 min to a stirred suspension with 31.99 g (84.4 mM) of 1-(3-cyclopentyloxy-4-methoxy-benzyl)-4,5-diamino-2-thiouracil in 315 ml of THF and 26.5 ml of triethylamine. After 2 hr at rt the solid was filtered off and the solution evaporated to dryness. The residue was treated with 400 ml of ether, 50 ml of saturated sodium bicarbonate solution and 100 ml of water. After 2 hr the crystals were collected and washed with ether and water: 30.14 g (66.3%) of amide. The ether phase was evaporated to dryness and the residue crystallized from methanol: 5.30 g (11.7%) of amide with mp 198-202°C.

### B. 8-(1-benzyloxy-1-methyl-ethyl)-3-(3-cyclopentyloxy-4-methoxy-benzyl)-2-thioxanthine

35.44 g (65.8 mM) of the above amide (A.) were added at 70°C to a solution with 29.53 g of t-BuOK in 350 ml of isopropanol and refluxed for 0.5 hr. The solvent was evaporated in vacuo, the residue dissolved in 350 ml of water, the solution treated twice with 3.0 g of charcoal and filtered. At 5°C the solution was neutralized with 56 ml of 5n HCl to pH 7. After 2.5 hr the solid was collected and suspended for 1.5 hr in 300 ml of methanol, cooled to 5°C and the solid collected again: 21.15 g (61.7%) of xanthine. The filtrate was evaporated in vacuo, the residue dissolved in dichloromethane and filtered through 60 g of silicagel in a column: crystallization from methanol gave another 5.75 g (16.8%) of xanthine with mp 158-160/235-237/288-290°C.

### C. 8-(1-benzyloxy-1-methyl-ethyl)-3-(3-cyclopentyloxy-4-methoxy-benzyl)-hypoxanthine

A solution with 22.39 g (43 mM) of the above xanthine (B.) in 580 ml of 1-propanol was treated with 25 g of Raney-nickel (washed with 0.1% aqueous acetic acid) and refluxed for 2 hr. The nickel was filtered off and the solvent evaporated in vacuo. The residue was crystallized from 120 ml of methanol: 12.73 g (60.6%) of hypo-xanthine with mp 161-162°C, a second crop gave 1.97 g (9.4%) of hypoxanthine.

### Example 6

In analogy to example 1 we prepared from 5,6-diamino-1-(3,4-dimethoxybenzyl)-2-thiouracil and 2-benzyloxy-2-methyl-propionyl chloride the following compounds:
A. 6-Amino-5-(2-benzyloxy-2-methyl-propionylamino)-1-(3,4-dimethoxybenzyl)-2-thiouracil
B. 8-(1-Benzyloxy-1-methyl-ethyl)-3-(3,4-dimethoxybenzyl)-2-thioxanthine
C. 8-(1-Benzyloxy-1-methyl-ethyl)-3-(3,4-dimethoxybenzyl)-hypoxanthine with mp 164-166°C

### Example 7

### 3-(3,4-Dimethoxybenzyl)-8-(1-(4-fluorobenzyloxy)-1-methyl-ethyl)-hypoxanthine

### A. 6-Amino-1-(3,4-dimethoxy-benzyl)5-(2-(4-fluorobenzyloxy)-2-methyl-propionylamino)-2-thiouracil

A solution with 3.70 g (15 mM) of 2-(4-fluorobenzyloxy)-2-methyl-propionyl chloride in 10 ml of THF was added at 0-5°C within 5 min to a suspension with 3.19 g (10.3 mM) of 5,6-diamino-1-(3,4-dimethoxybenzyl)-2-thiouracil and 3.5 ml of (25 mM) of triethylamine in 30 ml of THF. After 4 hr the solid was filtered off and the solvent evaporated in vacuo. The residue was dissolved in 40 ml of ethyl acatate and extracted with 20 ml of 1n HCl solution. A precipitate was formed and collected: 2.11 g (40.6%) of amide. The ethyl acetate phase was washed with sodium bicarbonate solution and evaporated to dryness: 3.70 g of a mainly two compound mixture. Separation of a dichloromethane solution, comprising 2% of methanol, on 60 g of silicagel in a column gave first after crystallization from acetone 1.11g (15.4%) of the diamide with mp 194-197°C and later fractions gave 1.06 g (20.4%) of amide with mp 110-180°C as a second crop.

### B. 3-(3,4-Dimethoxybenzyl)-8-(1-(4-fluorobenzyloxy-1-methyl-ethyl)-2-thioxanthine

A solution with 3.07 g (6.1 mM) of the above amide (A.) in 30 ml of 1n NaOH was refluxed for 15 min, treated twice with 0.2 of charcoal, filtered and neutralized with 6.2 ml of 5n HCl. The solid was collected, supended in 30 ml of hot methanol and collected again at 5°C: 2.34 g (79.1%) of xanthine with mp 300-302°C.

### C. 3-(3,4-Dimethoxybenzyl)-8-(1-(4-fluorobenzyloxy)-1-methyl-ethyl)-hypoxanthine

2.18 g (4.5 mM) of xanthine (B.) and 2.6 g of Raney-nickel (treated with 0.1% aqueous acetic acid) were refluxed in 30 ml of 1-propanol for 2.5 hr. The nickel was filtered off and the solution evaporated in vacuo to dryness. The residue was dissolved in 40 ml of ethyl acetate and extracted with sodium bicarbonate solution. The formed solid is filtered off and the organic phase evaporated in vacuo to dryness (1.95 g). A dichloromethane solution, comprising 2% of methanol , was purified on 19 g of silicagel in a column. Crystallization from ethyl acetate gave 1.37 g (67.2%) of hypoxanthine with mp 159-161°C, a second crop gave 0.4 g (2.0%) of hypoxanthine.

### Example 8

### 3-(3-Cyclopentyloxy-4-methoxy-benzyl)-8-(1-(4-methoxybenzyloxy)-1-methyl-ethyl)-hypoxanthine

### A. 6-Amino-1-(3-cyclopentyloxy-4-methoxy-benzyl)-5-(2-(4-methoxybenzyloxy)-2-methylpropionylamino)-2-thiouracil

A solution with 2.91 g (12 mM) of crude 2-(4-methoxybenzyloxy)-2-methyl-propionyl chloride in 9 ml of THF was added within 90 min to a stirred suspension with 3.62 g (10 mM) of 5,6-diamino-1-(3-cyclopentyloxy-4-methoxy-benzyl)-2-thiouracil. After 20 hr another 485 mg of chloride in 5 ml of THF was added within 30 min. After a further 3 hr the solid was filtered off and the solution evaporated in vacuo to dryness. The residue was dissolved in ethyl acetate, extracted with sodium bicarbonate solution and the organic phase evaporated in vacuo to dryness. The residue was crystallized from methanol: 1.71 g (30.1%) of amide with mp 172-184°C.

### B. 3-(3-Cyclopentyloxy-4-methoxy-benzyl)-8-(1-(4-methoxybenzyloxy)-1-methyl-ethyl)-2-thioxanthine

3.30 g (5.8 mM) of the above amide (A.) and 2.60 g of t-BuOK (23 mM) were refluxed in 33 ml of isopropanol for 45 min. The solvent was removed in vacuo, the residue dissolved in 50 ml of water, treated twice with 0.3 g of charcoal, filtered, acidified with 4.5 ml of 5n HCl to pH 4.5 and neutralized with sodium bicarbonate solution. The solid was collected and crystallized from methanol: 2.48 g (77.7%) of xanthine with mp 169/276-279°C.

### C. 3-(3-Cyclopentyloxy-4-methoxy-benzyl)-8-(1-(4-methoxybenzyloxy)-1-methyl-ethyl)-hypoxanthine

A solution with 2.34 g (4.25 mM) of the above xanthine (B.) and 2.5 g of Raney-nickel (treated with 0.1% of aqueous acetic acid) were refluxed in 35 ml of 1-propanol. After 2.5 hr the nickel was filtered off and the solvent evaporated in vacuo. The residue was dissolved in ethyl acetate, extracted with sodium bicarbonate solution and concentrated in vacuo to about 10 ml. Over night crystallization gave 1.29 g (58.6%) of hypoxanthine with mp 156-157°C.

### Example 9

In analogy to example 1 we prepared from 1-(3-cyclopentyloxy-4-methoxy-benzyl)-4,5-diamino-2-thiouracil and 2-(4-fluorobenzyloxy)-2-methyl-propionyl chloride the following compounds:
A. 6-Amino-1-(3-cyclopentyloxy-4-methoxy-benzyl)-5-(2-(4-fluorobenzyloxy)-2-methyl-propionylamino)-2-thiouracil with mp 185-193°C
B. 3-(3-Cyclopentyloxy-4-methoxy-benzyl)-8-(1-(4-fluorobenzyloxy)-1-methyl-ethyl)-2-thioxanthine with mp 188-192/288-296°C
C. 3-(3-Cyclopentyloxy-4-methoxy-benzyl)-8-(1-(4-fluorobenzyloxy)-1-methyl-ethyl)-hypoxanthine with mp 131-134°C

### Example 10

In analogy to example 1 we prepared from benzyloxyacetyl chloride and 1-(3-cyclopentyloxy-4-methoxy-benzyl)-4,5-diamino-2-thiouracil the following compounds:
A. 6-Amino-5-benzyloxyacetylamino-2-thiouracil with mp 195-196°C
B. 8-Benzyloxymethyl-3-(3-cyclopentyloxy-4-methoxy-benzyl)-2-thioxanthine with mp 101-103°C
C. 8-Benzyloxymethyl-3-(3-cyclopentyloxy-4-methoxy-benzyl)-hypoxanthine with mp 190-194°C

### Example 11

### 3-(3-Cyclopentyloxy-4-methoxy-benzyl)-8-isopropyl-hypoxanthine

4.15 g (10 mM) of 3-(3-Cyclopentyloxy-4-methoxy-benzyl)-8-isopropyl-2-thioxanthine was dissolved in 42 ml of 1N NaOH and treated with three portions of 3 g of Raney-nickel with 0.5 hour intervals. After a further 0.5 hour, the nickel was filtered off and the solution acidified with 8 ml of 5N HCl to pH 3 and then neutralized with sodium bicarbonate solution to pH 7. The solid was collected, washed and dried: 3.62 g (94.5%) of hypoxanthine having a melting point of 243-4°C.

### Example 12

### 3-(3-Cyclopentyloxy-4-methoxy-benzyl)-8-(1-hydroxy-1-methyl-ethyl)-hypoxanthine

### A. 6-Amino-5-(2-Hydroxy-2-methyl-propionylamino)-1-(3-cyclopentyloxy-4-methoxy-benzyl)-2-thiouracil

19.75 g (52 mM) of 1-(3-cyclopentyloxy-4-methoxy-benzyl)-5,6-diamino-2-thiouracil were added to 16.25 g (156 mM) of 98% 2-hydroxy-isobutyric acid finely suspended in 200 ml of 1,2-dimethoxy-ethane and refluxed for 42 hr. The yellow-orange suspension was cooled to 0°C and the solid was collected: 12.54 g ( 53.8%) of amide with mp 221-224°C.

### B. 3-(3-Cyclopentyloxy-4-methoxy-benzyl)-8-(1-hydroxy-1-methyl-ethyl)-2-thioxanthine

15.73 g (136 mM) of 97% potassium t-butylate were added to a suspension of 15.25 g (34 mM) of 6-amino-5-(2-hydroxy-2-methyl-propionylamino)-1-(3-cyclopentyloxy-4-methoxy-benzyl)-2-thiouracil in 250 ml of 2-propanol and refluxed for 50 min. The solvent was removed in vacuo, the residue dissolved in 200 ml of water, filtered and slowly acidified at 5°C with 31 ml of 5n HCl to pH 6. The solid was collected: 14.26 g (93.5%) of 2-thioxanthine with mp
(sublimation at 240°C) 270-274/C.

### C. 3-(3-Cyclopentyloxy-4-methoxy-benzyl-8-(1-hydroxy-1-methyl-ethyl)-hypoxanthine

14.8 g (126 mM) of 50% Raney-nickel was added in 8 portions to a solution of 2.47 g (5.5 mM) of 3-(3-cyclopentyloxy-4-methoxy-benzyl)-8-(1-hydroxy-1-methyl-ethyl)-2-thioxanthine in 30 ml of 1n NaOH with vigorous stirring. The nickel salt was filtered off, the solution acidified with 9 ml of 5n HCl to pH 6 and the solid collected and washed with water: 1.98 g (86.5%) of hypoxanthine with mp 243-245°C.

### Example 13

### 3-(3-Cyclopentyloxy-4-methoxy-benzyl)-8-(1-methly-ethenyl)-hypoxanthine

1.95 g (4.7 mM) of 3-(3-cyclopentyloxy-4-methoxy-benzyl)-8-(1-hydroxy-1-methyl-ethyl)-hypoxanthine in 30 ml of THF with 20 ml of acetic anhydride and 4 ml of triethylamine were heated to 60°C for 25 hr. After stirring over night at rt and cooling to 5°C the solid was collected and washed with THF: 1.36 g (76.0%) of title compound with mp 247-248°C.

| Elemental analyses for C₂₁H₂₄N₄O₃ / 380.44 | | | | |
|---|---|---|---|---|
| % calc | C 66.30 | H 6.36 | N 14.73 | O 12.62 |
| % found | C 65.84 | H 6.70 | N 14.15 | O 12.80 |

### Example 14

### 3-(3,4-Methylenedioxy-benzyl)-8-(1-methyl-ethyl)-hypoxanthine

### A. N-(3,4-Methylenedioxy-benzyl)-thiourea

A fine suspension of 42.4 g (226 mM) of 3,4-methylenedioxy-benzylamine hydrochloride and 34.67 g (357 mM) of potassium thiocyanate in 300 ml of toluene was refluxed for 23 hr with a water collector. The solid was collected, suspended in 250 ml of water, adjusted to pH 8 with saturated sodium bicarbonate solution, the solid collected again and washed with water: 5.45 g (11.5%) of thiourea with mp 161-162°C. 71.4% of amine was recovered.

### B. 6-Amino-1-(3,4-methylenedioxy-benzyl)-2-thiouracil

30.91 g (147 mM) of N-(3,4-methylenedioxy-benzyl)-thiourea were added to a solution of 18.7 g (168 mM) of 97% potassium t-butylate in 170 ml of 2-propanol and at 70°C followed by 16 ml of ethyl cyanoacetate. After 6 hr at reflux (after 30 min formation of a solution) at 70°C another batch with 1.70 g (14.7 mM) of butylate and 3.2 ml (30 mM) of cyanoacetate was added. After a total of 23 hr at refux the formed suspension was collected at rt. The solution was evaporated in vacuo, the residue dissolved in 700 ml of water and the collected solid added again. The suspension was neutralized to pH 7.5 with 23 ml of 5n HCl, the solid collected and washed with 200 ml of 0.2n sodium bicarbonate solution and water. The dried solid was suspended in 600 ml of refluxing ether for 30 min, concentrated to 450 ml and collected again: 39.60 g (97.1%) of thiouracil with mp 241-244°C.

### C. 6-Amino-1-(3,4-methylenedioxy-benzyl)-5-(2-methyl-propionylamino)-2-thiouracil

10.6 ml (156.7 mM) of 85% phosphoric acid and within 20 min 37.4 ml (149.6 mM) of 4n sodium nitite solution were added at 55° to 60°C to a stirred suspension of 39.5 g (142.5 mM) of 4-amino-3-(3,4-methylenedioxy-benzyl)-2-thiouracil in 1.00 1 of THF. After a further 10 min 58.4 g of 85% sodium dithionate suspended in 375 ml of water were added at 30°C. The green suspension turned within 10 min to a yellow two-phase solution. After a further 5 min 35.5 ml (213.8 mM) of isobutyric anhydride were added at rt and stirred over night. The newly formed suspension was neutralized with 490 ml of 2n NaOH to pH 8 and after 2 hr the solid was collected. The filtrate was concentrated in vacuo until only water distilled off and the solid collected again: total amount 41.89 g (81.1%) of amide with mp 236-237°C.

### D. 3-(3,4-Methylenedioxy-benzyl)-8-(1-methyl-ethyl)-2-thioxanthine

41.82 g (115.4 mM) of 6-amino-1-(3,4-methylenedioxy-benzyl)-5-(2-methyl-propionylamino)-2-thiouracil were refluxed in 400 ml of 1n NaOH for 30 min, treated twice with 5 g of charcoal, filtered, the solution neutralized at 5°C with 87 ml of 5n HCl to pH 7, after a further 30 min the solid was collected, washed, suspended in 550 ml of hot methanol for 30 min, concentrated to 400 ml and the solid collected again at about 40°C: 29.71 g ( 74.8%) of thioxanthine with mp 320°C.

### E. 3-(3,4-Methylenedioxy-benzyl)-8-(1-methyl-ethyl)-hypoxanthine

70 g (1.19 M) of Raney-nickel were added in 7 portions at rt to a solution of 25.83 g (75 mM) of 3-(3,4-methylenedioxy-benzyl)-8-(1-methyl-ethyl)-2-thioxanthine in 300 ml of 1n NaOH. The nickel was filtered off, the solution acidified with 61.5 ml of 5n HCl to pH 4.5 and neutralized with saturated sodium bicarbonate solution to pH 7. After 1 hr the solid was collected at 5°C and washed with water: 20.68 g (88.3%) of hypoxanthine, which was suspended for 1 hr in 200 ml of hot THF, concentrated to 150 ml and the solid was collected again at 5°C: 20.02 g (85.5%) of hypoxanthine with mp 254-257°C.

### Example 15

### 3-(3,4-Dimethoxy-benzyl)-8-(1-methyl-ethyl)-hypoxanthine

### A. N-(3,4-Dimethoxy-benzyl)-thiourea

28.54 g/22.65 ml (375 mM) of carbon disulfide were added with cooling to a stirred solution of 10 g (250 mM) of NaOH in 35 ml of water, within 25 min a solution 39.1 ml (250 mM) of 95% veratrylamine in 20 ml of water were added and the mixture was heated to 75 °C within 20 min and the solution was kept for 1 hr at 75 to 80°C. After cooling to 45°C 23.8 ml (250 mM) of ethyl chloroformate were added slowly. Within 30 min 8 ml of 2n NaOH were added dropwise, the pH elevated from 6 to 8 and the formation of COS started. After about 1 hr the gas formation ceded and after a further 30 min 150 ml of ether were added to dilute the lower mustard oil phase. The now upper ether phase was collected and washed with half saturated NaCl solution. 58.8 g (112%) of isothiocyanate which was dissolved in 60 ml of THF and treated with 30.2 ml of 32% aqueous ammonia solution (exothermic). After 45 min the solvents were removed in vacuo and the residue suspended in 100 ml of water. The solid was collected (56.2 g), suspended in 130 ml of hot acetone and the solid collected again at 5°C: 49.37 g (87.3%) of thiourea with mp 192-193°C.

### B. 4-Amino-3-(3,4-dimethoxy-benzyl)-2-thiouracil

49.33 g (218 mM) of N-(3,4-dimethoxy-benzyl)-thiourea were refluxed in a solution of 27.74 g (240 mM) of 97% potassium t-butylate in 200 ml of 2-propanol for dissolution and below refluxing temperature 23.7 ml (222 mM) of ethyl cyanoacetate were added. After 6 hr at reflux a second batch of 2.52 g (21.8 mM) of butylate and 4.65 ml (43.6 mM) of cyanaoacetate were added below refluxing. After refluxing over night the solvent was evaporated in vacuo, the residue dissolved in 600 ml of water, concentrated to 500 ml and neutralized to pH 7.5 with 40 ml of 5n HCl with concomittant dilution with 800 ml of water. The solid was collected, washed with 250 ml of 0.2n sodium bicarbonate solution and 400 ml of water, resuspended in 300 ml of hot methanol, concentrated to 250 ml and collected again at 5°C: 52.39 g (81.9%) of thiouracil with mp (sublimation at 205°C) 234-235°C.

### C. 6-Amino-1-(3,4-dimethoxy-benzyl)-5-nitroso-2-thiouracil

39.5 ml (158 mM) of 4n sodium nitrite solution were added at 65-70°C within 10 min to a solution of 45.47 g (155 mM) of 4-amino-3-(3,4-dimethoxy-benzyl)-2-thiouracil in 900 ml of acetic acid. After a further 10 min 900 ml of water were added at 40°C within 30 min. The solid was collected and washed with 200 ml of acetic acid/water 1:1 and 250 ml of water: 44.6 g of crystals which were dissolved in 600 ml of 1n NaOH, filtered and acidified with 114 ml of 5n HCl to pH 4. The solid was collected again at 10°C and washed with 700 ml of water:
42.03 g (84.1%) of nitrosouracil.

### D. 6-Amino-1-(3,4-dimethoxy-benzyl)-5-(2-methyl-propionylamino)-2-thiouracil

53.25 g (260 mM) of sodium dithionate were added in portions to a stirred suspension of 41.90 g (130 mM) of 6-amino-1-(3,4-dimethoxy-benzyl)-5-nitroso-2-thiouracil in 400 ml of THF and 150 ml of water (exothermic). After 30 min at 35-40°C 32.4 ml (195 mM) of isobutyric anhydride were added and stirred over night. The THF was removed in vacuo, the suspension diluted with 200 ml of water and the pH reduced to 6 with 130 ml of 2n NaOH and further to pH 7.5 with 33.7 g of sodium bicarbonate (CO₂ formation). The solid was collected at 10°C and washed with 250 ml of water: 43.53 g (88.5%) of amide.

### E. 3-(3,4-Dimethoxy-benzyl)-8-(1-methyl-ethyl)-2-thioxanthine

43.54 g (115 mM) of 6-amino-1-(3,4-dimethoxy-benzyl)-5-(2-methyl-propionylamino)-2-thiouracil were refluxed in 450 ml of 1n NaOH for 30 min. The insoluble part at rt was filtered off (1.57 g of thiourea), the solution was acidified with 85 ml of 5n HCl to pH 4 and neutralized with saturated sodium bicarbonate solution to pH 7. The solid was collected at 10°C and washed with 400 ml of water: 37.08 g of crude thioxanthine which was suspended in 600 ml of hot methanol, collected again at rt: 31.02 g which were resuspended in 500 ml of hot acetone and collected again: 30.34 g. The crystals were dissolved in 300 ml of 1n NaOH, treated twice with 3.0 g of charcoal, filtered, diluted with 150 ml of methanol and neutralized with 11.7 ml of 85% phosphoric acid in 90 ml of water. The solid was collected and washed with 250 ml of water: 29.29 g (70.7%) of thioxanthine with mp 240°C sublimation at 260°-290°C.

### F. 3-(3,4-Dimethoxy-benzyl)-8-(1-methyl-ethyl)-hypoxanthine

70 g Raney-nickel were added in 7 portions to a stirred solution of 25.23 g (70 mM) of 3-(3,4-dimethoxy-benzyl)-8-(1-methyl-ethyl)-2-thioxanthine in 300 ml of 1n NaOH. The nickel was filtered off, the solution acidified to pH 4 with 58 ml of 5n HCl and neutralized with saturated sodium bicarbonate solution to pH 7. The solid was collected, washed with 250 ml of water, suspended in 200 ml of hot THF, concentrated to 150 ml and collected again at 5 °C: 20.58 g (89.5%) of hypoxanthine, containing THF, with mp 216-218°C.

### Example 16

### 3-(3-Hydroxy-4-methoxy-benzyl)-8-(1-methyl-ethyl)-hypoxanthine

9.0 g of Raney-nickel were added in 9 portions with stirring to a solution of 0.87 g (2.0 mM) of 3-(3-benzyloxy-4-methoxy-benzyl)-8-(1-methyl-ethyl)-2-thioxanthine in 20 ml of 0.55n NaOH. The nickel was filtered off and the solution neutralized with 6 ml of 2n HCl to pH 6.5. The solid was collected and washed with water: 0.63 g (94.6%) of hypoxanthine hydrate with mp 230°C with decomposition.

### Example 17

Following the previously set forth methods of examples 1 or 11 the following hypoxanthines and thiohypoxanthines of the present invention were synthesized from the corresponding 2-thioxanthines. The chemical name and mp are provided below.

### A. 3-(4-Methoxy-benzyl)-8-(1-methyl-ethyl)-hypoxanthine

(94.6%) with mp (sublimation at 243°C) 258-270°C

### B. 3-(3-Chloro-benzyl)-8-(1-methyl-ethyl)-hypoxanthine

(63.2%) with mp 237-240°C

### C. 3-(4-Chloro-benzyl)-8-(1-methyl-ethyl)-hypoxanthine

(83.6%) with mp 278-280°C

### D. 3-(2-Methyl-butyl)-hypoxanthine

(65.7%) with mp 187-193 °C
3-(2-Methyl-butyl)-thiohypoxathine
(96.5%) with mp 220°C, sublimation 297-313°C with decomposition

### E. 3-(3-Methyl-butyl)-hypoxanthine

(70.0%) with mp 199-202°C
3-(3-Methyl-butyl)-thiohypoxanthine
(52.4%) with mp 215°C, sublimation 250-270°C

### F. 3-Benzyl-hypoxanthine

(88.2%) with mp 230° sublimation at 268-274°C
3-Benzyl-thiohypoxanthine
only crude

### G. 3-Hexyl-hypoxanthine

(60%) with mp 184-186°C
3-Hexyl-thiohypoxanthine
(25.5%) with mp 210°C, sublimation 280-288°C with decomposition

### H. 8-Cyclopropyl-3-ethyl-hypoxanthine

(80.8%) with mp 200°C, sublimation 300°C
8-Cyclopropyl-3-ethyl-thiohypoxanthine
(31.5%) with mp220°C sublimation 250-270°C with decomposition

### I. 3-Ethyl-hypoxanthine

(50.9%) with mp 210°C, sublimation 272-273°C
3-Ethyl-thiohypoxanthine
(41.9%) with mp 220°C, sublimation 285-304°C with decomposition

### J. 3-Butyl-hypoxanthine

(36.4%) with mp 187-193°C

### 3-Butyl-thiohypoxanthine

(63.7%) with mp 220°C, sublimation 245-295°C with decomposition

### Enzyme Isolation Protocols

Protocols for obtaining PDE III, PDE IV and PDE V, and measuring inhibition activities are set forth below:

### Type III Phosphodiesterase

Protocol for Enzyme Isloation: The Type III PDE is isolated from human platelets using a procedure similar to that previously described by Weishaar, R.E.; Burrows, S.D.; Kobylarg, D.C., Quade, N.M.; Evans, D.B., Biochem. Pharmacol., 35:787, 1986. Briefly, 1-2 units of platelets are suspended in an equal volume of buffer (20 mM Tris-HCl, pH 7.5, containing 2 mM magnesium acetate, 1 mM dithiothreitol, and 5 mM Na₂ EDTA). The protease inhibitor phenylmethyl-sulfonyl fluoride (PMSF) is also included in this buffer at a final concentration of 200 mM. The suspension is homogenized using a polytron and the homogenate centrifuged at 100,000 x g for 60 minutes. This and all subsequent procedures are performed at 0-4°C. The supernatant is then filtered through four layers of gauze and applied to a DEAE-Trisacryl M column, previously equilibrated with buffer B (20 mM Tris-HCl, pH 7.5, containing 1 mM magnesium acetate, 1 mM dithiothreitol and 200 mM PMSF). After application of the sample, the column is washed with several bed volumes of buffer B, after which the different forms of PDE are eluted from the column using two successive linear NaCl gradients (0.05-0.15 M, 300 ml total; 0.15-0.40 M, 200 ml total). Five milliliter fractions are collected and assayed for cyclic AMP and cyclic GMP PDE activity. Fractions containing PDE III activity are pooled and dialyzed overnight against 4 liters of buffer B. The dialyzed PDE III is then concentrated to 10% of the original volume, diluted to 50% with ethylene glycol monoethyl ether and stored at -20°C. PDE III can typically be retained for up to four weeks with little or no loss of activity.

Measuring Type III PDE Activity: Enzyme activity is assessed by measuring the hydrolysis of [¹H]-cyclic AMP, as described by Thompson, W.J., Teraski, W.L., Epstein, P.N., Strada. S.J.: Adv. Cyclic Nucleotide Res. 10:69, 1979. The cyclic AMP concentration used in this assay is 0.2 mM, which approximates to the Kₘ value. Protein concentration is adjusted to ensure that no more than 15% of the available substrate is hydrolyzed during the incubation period.

All test compounds are dissolved in dimethyl sulfoxide (final concentration of 2.5%). This concentration of dimethyl sulfoxide inhibits enzyme activity by approximately 10%.

### Type IV Phosphodiesterase

Protocol for Enzyme Isolation: The Type IV PDE is isolated from bovine tracheal smooth muscle using a procedure similar to that previously described by Silver, P.J., et al.: Eur. J. Pharmacol. 150:85, 1988.(1). Briefly, smooth muscle from bovine trachea is minced and homogenized using a polytron in 10 volumes of an extraction buffer containing 10 mM Tris-acetate (pH 7.5), 2 mM magnesium chloride, 1 mM dithiothreitol and 2,000 units/ml of aprotinin. This and all subsequent procedures are performed at 0-4°C. The homogenate is sonicated and then centrifuged at 48,000 x g for 30 minutes. The resulting supernatant is applied to a DEAE Trisacryl M column previously equilibrated with sodium acetate and dithiothreitol. After applications of the sample, the column is washed with sodium acetate/dithiothreitol, after which the different forms of PDE are eluted from the column using a linear Tris-HCl/NaCl gradient. Fractions containing Type IV PDE are collected, dialyzed and concentrated to 14% of the original volume. The concentrated fractions are diluted to 50% with ethylene glycol and stored at -20°C.

Measuring Type IV PDE Activity Enzyme activity is assessed by measuring the hydrolysis of [³H]-cyclic AMP, as described by Thompson, W.J., et al.: Adv. Cyclic Nucleotide Res. 10:69, 1979. The cyclic AMP concentration used in this assay is 0.2 mM, which approximates to the Kₘ value. Protein concentration is adjusted to ensure that no more than 15% of the available substrate is hydrolyzed during the incubation period.

All test compounds are dissolved in dimethyl sulfoxide (final concentration of 2.5%). This concentration of dimethyl sulfoxide inhibits enzyme activity by approximately 10%.

### Measuring Type V PDE Activity

Protocol for Enzyme Isloation: The Type V PDE is isolated using a procedure similar to that previously described by Weishaar et al., Hypertension 15:528, (1990). Briefly, 1-2 units of platelets are suspended in an equal volume of buffer A (20 mM Tris-HCl, pH 7.5, containing 2 mM magnesium acetate, I mM dithiothreitol, and 5 mM Na₂EDTA) using a polytron. The proteinase inhibitor phenylmethylsulfonyl fluoride (PMSF) are also included in this buffer at a final concentration of 200 uM. This and all subsequent procedures are performed at 0-4°C. The homogenate is then centrifuged at 100,000 rpm for 60 minutes. The supernatant is then removed and filtered through four layers of gauze and applied to a DEAE-Trisacryl M column. The column is washed with several bed volumes of buffer B (20 mM Tris-HCl, pH 7.5, containing 2 mM magnesium acetate, 1 mM dithiothreitol, and 200 mM PMSF) and eluted by two successive linear NaCl gradients (0.05-0.15 M, 300 ml total; 0.15-0.40 M, 200 ml total). Five ml fractions are collected and assayed for cyclic AMP and cyclic GMP PDE activity. Fractions that contain PDE V are pooled and dialyzed overnight against 4 L of buffer C (20 mM Tris-HCl, pH 7.5, containing 2 mM magnesium acetate and proteinase inhibitors). The dialyzed PDE V is then concentrated to 10% of the original volume, diluted to 50% with ethylene glycol monoethyl ether and stored at -20°C. PDE V can typically be retained for up to four weeks with little or no loss of activity.

Measuring Type V PDE Activity: Enzyme activity is assessed by measuring the hydrolysis of [³H]-cyclic GMP, as described by Thompson et al. (Thompson, W.J., Teraski, W.L., Epstein, P.N., Strada, S.J.: Adv. Cyclic Nucleotide Res. 10:69, 1979). The cyclic GMP concentration used in this assay is 0.2 uM, which approximates to the Kₘ value. Protein concentration is adjusted to ensure that no more than 15% of the available substrate is hydrolyzed during the incubation period.

All test compounds are dissolved in dimethyl sulfoxide (final concentration of 2.5%). This concentration of dimethyl sulfoxide inhibits enzyme activity by approximately 10%. The reference Type V PDE inhibitor zaprinast is evaluated with each assay.

The compounds are tested over concentration range: 0.1, 1, 10, 100 uM (n=1), and IC₅₀ determinations are made using 5 appropriate concentrations (n=2).

As can be seen from the foregoing, the compositions of the present invention are also potent inhibitors of PDE V in mammals. Such activity is useful in the medical arts to reduce smooth muscle cell proliferation and increase pulmonary vasodilation. In certain aspects of the invention, the compounds demonstrate a combination of selective PDE IV and PDE V inhibition and can be used in diseases such as restenosis and related diseases. Such aspects, of course, include administering an effective amount of a compound of the present invention possessing said combination of PDE IV and V inhibitory activities to a mammal in need of such therapy.

Following the above procedures, the PDE III, PDE IV and PDE V inhibition for the following compounds were tested. The results are set forth in table I below.

**TABLE I**

| EXAMPLE | PDE III IC₅₀(:M) | PDE IV IC₅₀(:M) | PDE V IC₅₀(micro M) |
|---|---|---|---|
| 3-(3-cyclopentyloxy-4-methoxy-benzyl)-8-(1-hydroxy-1-methyl-ethyl)-hypoxanthine | | 0.39 | 193.370 |
| 3-(3-cyclopentyloxy-4-methoxy-benzyl)-8-(1-benzyloxy-1-methyl-ethyl)-hypoxanthine | 19.78 | 0.07 | 8.280 |
| 3-(3-cyclopentyloxy-4-methoxy-benzyl)-8-(benzyloxymethyl)-hypoxanthine | 3.89 | 0.157227 | 7.527 |
| 3-(3-cyclopentyloxy-4-methoxy-benzyl)-8-(1-methyl-ethyl)-hypoxanthine | 41.90 | 0.4 | 49.000 |
| 3-(3-cyclopentyloxy-4-methoxy-benzyl)-8-(1-(4-methoxybenzyloxy)-1-methyl-ethyl)-hypoxanthine | | 0.075 | |
| 3-(3-cyclopentyloxy-4-methoxy-benzyl)-8-(1-(4-fluorobenzyloxy)-1-methyl-ethyl)-hypoxanthine | 32.80 | 0.000343 | 14.481 |
| 3-(3-benzyloxy-4-methoxy-benzyl)-8-(1-benzyloxy-1-methyl-ethyl)-hypoxanthine | 57.65 | 6.38 | 12.470 |
| 3-(3-4-dimethoxy-benzyl)-8-(1-benzyloxy-1-methyl-ethyl)-hypoxanthine | 90.26 | 0.66 | 10.000 |
| 3-(3-benzyloxy-4-methoxy-benzyl)-8-(1-methyl-ethyl)-hypoxanthine | | 10.63 | |
| 3-(3-hydroxy-4-methoxy-benzyl)-8-(1-methyl-ethyl)-hypoxanthine | | 69.42 | |
| 3-(3-4-dimethoxy-benzyl)-8-(1-methyl-ethyl)-hypoxanthine | 63.58 | 2.47947 | 10.586 |
| 3-(1,3-benzodioxole-5-methyl)-8-(1-methyl-ethyl)-hypoxanthine | | 33.46 | |
| 3-(4-chloro-benzyl)-8-(1-methyl-ethyl)-hypoxanthine | 148.50 | 34.1 | 7.600 |
| 3-(3-chloro-benzyl)-8-(1-methyl-ethyl)-hypoxanthine | 336.78 | 53.64699 | 6.315 |
| 3-(4-methoxy-benzyl)-8-(1-methyl-ethyl)-hypoxanthine | | 37.7072 | |
| 3-(3-4-dimethoxy-benzyl)-8-(1-(4-fluorobenzyloxy)-1-methyl-ethyl)-hypoxanthine | 69.62 | 1.079 | 35.800 |

While the invention has been illustrated with respect to the production and use of particular compounds, it is apparent that variations and modifications of the invention can be made without departing from the scope of the invention.

## Claims

1. A pharmaceutical composition comprising a compound of the formula: 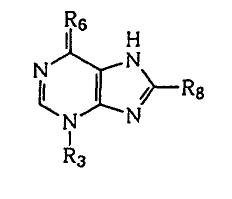 wherein; .
R₆ = S or O
R₃ is selected from the group consisting of C₁ - C₈ linear or branched alkyl; C₂ - C₈ linear or branched alkene; C₂ - C₈ linear or branched alkyne; C₃₋₈ cycloalkyl; Q, and K;
R₈ is selected from the group consisting of C₁ - C₈ linear or branched alkyl; C₂ - C₈ linear or branched alkene; C₂ - C₈ linear or branched alkyne; C₃₋₈ cycloalkyl; Q; and K;
wherein
Q has the general formula: 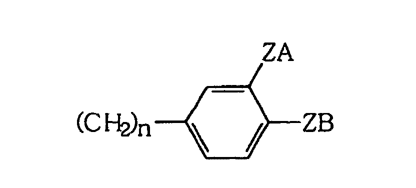 wherein;
n = 0 or 1;
Z = a bond, CH₂, NH, O or S;
A and B can form a ring by adding 1-3 CH₂ groups when Z = CH2, NH, O or S; and
A and B are not in a ring when Z = a bond, wherein A and B are independently selected from the group consisting of hydrogen; halogen; C₁ - C₈ alkyl; C₁ - C₈ alkoxy; C₃ - C₈ cycloalkoxy; hydroxy; phenyl; benzyl; and benzyloxy; wherein said phenyl, benzyl and benzyloxy are optionally substituted with halogen, C₁ - C₈ alkyl, C₁ - C₈ alkoxy, C₃ - C₈ cycloalkyl, C3 - C8 cycloalkoxy and hydroxy;
K has the general formula: 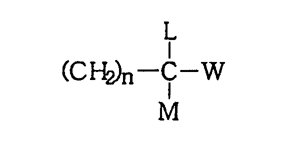 wherein;
n = 0 or 1;
L and M are independently selected from the group consisting of hydrogen and methyl;
W is selected form the group consisting of Q; hydroxy; benzyloxy optionally substituted with halogen, C1 - C8 alkyl, C1 - C8 alkoxy, C3 - C8 cycloalkyl, C3 - C8 cycloalkoxy and hydroxy; aryl; heteroaryl; and a heterocyclic ring;
provided that when R₃ is methyl, R8 is not hydrogen;
and pharmaceutically acceptable salts thereof;
and at least one pharmaceutically acceptable excipient; said composition in the form of a solid dosage form selected from the groups consisting of a tablet, gelcap, caplet, granule and lozenge.

2. The pharmaceutical composition of claim 1 wherein R₃ is benzyl.

3. The pharmaceutical composition of claim 1 wherein R₃ is benzyl substituted with an alkoxy and a cycloalkoxy group.

4. The pharmaceutical composition of claim 1 wherein R₃ is benzyl substituted with benzyloxy.

5. The pharmaceutical composition of claim 1 which is selected from the group consisting of:
3-butyl-hypoxanthine;
3-butyl-thiohypoxanthine;
3-ethyl-hypoxanthine
3-ethyl-thiohypoxanthine
3,8-diethyl-hypoxanthine;
3,8-diethyl-thiohypoxanthine;
3-ethyl-8-cyclopropyl-hypoxanthine;
3-ethyl-8-cyclopropyl-thiohypoxanthine;
3-propyl-hypoxanthine;
3-hexyl-hypoxanthine;
3-hexyl-thiohypoxanthine;
3-benzyl-hypoxanthine;
3-benzyl-thiohypoxanthine;
3-(4-methyl-butyl)-hypoxanthine;
3-(4-methyl-butyl)-thiohypoxanthine;
3-(2-methyl-butyl)-hypoxanthine;
3-(2-methyl-butyl)-thiohypoxanthine;
3-(3-cyclopentyloxy-4-methoxy-benzyl)-hypoxanthine;
3-(3-cyclopentyloxy-4-methoxy-benzyl)-8(1-hydroxy-1-methyl-ethyl)-hypoxanthine;
3-(3-cyclopentyloxy-4-methoxy-benzyl)-8(1-methyl-ethylene)-hypoxanthine;
3-(3-cyclopentyloxy-4-methoxy-benzyl)-8(1-benzyloxy-1-methyl-ethyl)-hypoxanthine;
3-(3-cyclopentyloxy-4-methoxy-benzyl)-8-(benzyloxymethyl)-hypoxanthine;
3-(3-cyclopentyloxy-4-methoxy-benzyl)-8-(1-methyl-ethyl)-hypoxanthine;
3-(3-cyclopentyloxy-4-methoxy-benzyl)-8-(1-(4-methoxybenzyloxy)-1-methyl-ethyl-hypoxanthine;
3-(3-cyclopentyloxy-4-methoxy-benzyl)-8-(1-(4-fluorobenzyloxy)-1-methyl-ethyl)-hypoxanthine;
3-(3-cyclopentyloxy-4-methoxy-benzyl)-8-(1-benzyloxy-1-methyl-ethyl)-hypoxanthine;
3-(3-4-dimethoxy-benzyl)-8-(1-benzyloxy-1-methyl-ethyl)-hypoxanthine;
3-(3-benzyloxy-4-methoxy-benzyl)-8-(1-methyl-ethyl)-hypoxanthine;
3-(3-hydroxy-4-methoxy-benzyl)-8-(1-methyl-ethyl)-hypoxanthine;
3-(3-4-dimethoxy-benzyl)-8-(1-methyl-ethyl)-hypoxanthine;
3-(1,3-benzodioxole-5-methyl)-8(1-methyl-ethyl)-hypoxanthine;
3-(4-chloro-benzyl)-8-(1-methyl-ethyl)-hypoxanthine;
3-(3-chloro-benzyl)-8-(1-methyl-ethyl)-hypoxanthine;
3-(4-methoxy-benzyl)-8-(1-methyl-ethyl)-hypoxanthine;
3-(3-4-dimethoxy-benzyl)-8-(1-(4-fluorobenzyloxy)-1-methyl-ethyl)-hypoxanthine;
and pharmaceutically acceptable salts thereof.

6. A method for preparing a pharmaceutical composition according to one of claims 1-5 for the treatment of a mammal suffering from a disease state selected from the group consisting of asthma, allergies, inflammation, depression, dementia and disease states associated with abnormally high physiologic levels of cytokines.

## Patentansprüche

1. Pharmazeutische Zusammensetzung, die einer Verbindung entsprechend der folgenden Formel, 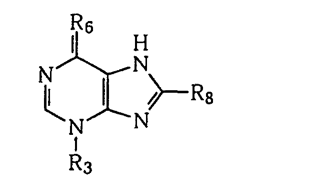 wobei
R₆ = S oder O ist;
R₃ ausgewählt ist aus der Gruppe bestehend aus geradkettigem oder verzweigtem C₁ - C₈ Alkyl; geradkettigem oder verzweigtem C₂ - C₈ Alken; geradkettigem oder verzweigtem C₂ - C₈ Alkin; C₃₋₈ Cycloalkyl; Q und K;
R₈ ausgewählt ist aus der Gruppe bestehend aus geradkettigem oder verzweigtem C₁ - C₈ Alkyl; geradkettigem oder verzweigtem C₂ - C₈ Alken; geradkettigem oder verzweigtem C₂ - C₈ Alkin; C_{3 - 8} Cycloalkyl; Q und K;
wobei
Q der allgemeinen Formel 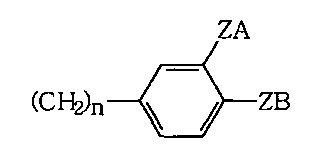 entspricht,
wobei
n = 0 oder 1 ist;
Z = eine Bindung, CH₂, NH, O oder S ist;
A und B durch Hinzufügen von 1-3 CH₂ Gruppen einen Ring bilden können, wenn Z = CH₂, NH, O oder S ist; und
A und B nicht in dem Ring sind, wenn Z = eine Bindung ist, wobei A und B unabhängig aus der Gruppe, bestehend aus Wasserstoff; Halogen; C₁ - C₈ Alkyl; C₁ - C₈ Alkoxy; C₃ -C₈ Cycloalkoxy; Hydroxy; Phenyl; Benzyl und Benzyloxy, ausgewählt sind, wobei besagtes Phenyl, Benzyl und Benzyloxy optional mit Halogen, C₁ - C₈ Alkyl, C₁ - C₈ Alkoxy, C₃ - C₈ Cycloalkyl, C₃ - C₈ Cycloalkoxy und Hydroxy substituiert ist;
K der allgemeinen Formel 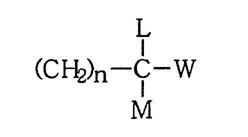 entspricht,
wobei
n = 0 oder 1 ist;
L und M unabhängig aus der Gruppe, bestehend aus Wasserstoff und Methyl, ausgewählt sind;
W aus der Gruppe, bestehend aus Q; Hydroxy; optional mit halogensubstituiertem Benzyloxy, C₁ - C₈ Alkyl, C₁ - C₈ Alkoxy, C₃ - C₈ Cycloalkyl, C₃ - C₈ Cycloalkoxy und Hydroxy; Aryl; Heteroaryl und einem heterozyklischen Ring ausgewählt ist,
mit der Maßgabe, dass, falls R₃ gleich Methyl ist, R₈ ungleich Wasserstoff ist;
bzw. ein pharmazeutisch akzeptables Salz einer solchen Verbindung, sowie mindestens einen pharmazeutischen Arzneiträger umfasst;
wobei diese Zusammensetzung in Form einer festen Dosierungsform vorliegt, ausgewählt aus der Gruppe, bestehend aus einer Tablette, Gelkapsel, Kapsel, Granule und Pastille.

2. Die pharmazeutische Zusammensetzung gemäß Anspruch 1, wobei R₃ Benzyl ist.

3. Die pharmazeutische Zusammensetzung gemäß Anspruch 1, wobei R₃ Benzyl ist, das mit einer Alkoxy und einer Cycloalkoxy-Gruppe substituiert ist.

4. Die pharmazeutische Zusammensetzung gemäß Anspruch 1, wobei R₃ ein Benzyloxy-substituiertes Benzyl ist.

5. Die pharmazeutische Zusammensetzung gemäß Anspruch 1, ausgewählt aus der Gruppe bestehend aus:
3-Butyl-hypoxanthin;
3-Butyl-thiohypoxanthin;
3-Ethyl-hypoxanthin
3-Ethyl-thiohypoxanthin
3,8-Diethyl-hypoxanthin;
3,8-Diethyl-thiohypoxanthin;
3-Ethyl-8-cyclopropyl-hypoxanthin;
3-Ethyl-8-cyclopropyl-thiohypoxanthin;
3-Propyl-hypoxanthin;
3-Hexyl-hypoxanthin;
3-Hexyl-thiohypoxanthin;
3-Benzyl-hypoxanthin;
3-Benzyl-thiohypoxanthin;
3-(4-Methyl-butyl)-hypoxanthin;
3-(4-Methyl-butyl)-thiohypoxanthin;
3-(2-Methyl-butyl)-hypoxanthin;
3-(2-Methyl-butyl)-thiohypoxanthin;
3-(3-Cyclopentyloxy-4-methoxy-benzyl)-hypoxanthin;
3-(3-Cyclopentyloxy-4-methoxy-benzyl)-8(1-hydroxy-1-methyl-ethyl)-hypoxanthin;
3-(3-Cyclopentyloxy-4-methoxy-benzyl)-8(1-methyl-ethylene)-hypoxanthin;
3-(3-Cyclopentyloxy-4-methoxy-benzyl)-8(1-benzyloxy-1-methyl-ethyl)-hypoxanthin;
3-(3-Cyclopentyloxy-4-methoxy-benzyl)-8-(benzyloxymethyl)-hypoxanthin;
3-(3-Cyclopentyloxy-4-methoxy-benzyl)-8-(1-methyl-ethyl)-hypoxanthin;
3-(3-Cyclopentyloxy-4-methoxy-benzyl)-8-(1-(4-methoxybenzyloxy)-1-methyl-ethyl-hypoxanthin;
3-(3-Cyclopentyloxy-4-methoxy-benzyl)-8-(1-(4-fluorobenzyloxy)-1-methylethyl)-hypoxanthin;
3-(3-Cyclopentyloxy-4-methoxy-benzyl)-8-(1-benzyloxy-1-methyl-ethyl)-hypoxanthin;
3-(3-4-Dimethoxy-benzyl)-8-(1-benzyloxy-1-methyl-ethyl)-hypoxanthin;
3-(3-Benzyloxy-4-methoxy-benzyl)-8-(1-methyl-ethyl)-hypoxanthin;
3-(3-Hydroxy-4-methoxy-benzyl)-8-(1-methyl-ethyl)-hypoxanthin;
3-(3-4-Dimethoxy-benzyl)-8-(1-methyl-ethyl)-hypoxanthin;
3-(1,3-Benzodioxole-5-methyl)-8(1-methyl-ethyl)-hypoxanthin;
3-(4-Chloro-benzyl)-8-(1-methyl-ethyl)-hypoxanthin;
3-(3-Chloro-benzyl)-8-(1-methyl-ethyl)-hypoxanthin;
3-(4-Methoxy-benzyl)-8-(1-methyl-ethyl)-hypoxanthin;
3-(3-4-Dimethoxy-benzyl)-8-(1-(4-fluorobenzyloxy)-1-methyl-ethyl)-hypoxanthin;
und pharmazeutisch verträgliche Salze derer.

6. Ein Verfahren zur Herstellung einer pharmazeutischen Zusammensetzung gemäß einem der Ansprüche 1-5 zur Behandlung eines Säugetiers, das unter einem Krankheitszustand leidet, der aus der Gruppe bestehend aus Asthma, Allergien, Entzündung, Depression, Demenz und Krankheitszuständen, die mit einem abnormalen hohen physiologischen Level an Cytokinen verbunden sind, ausgewählt ist.

## Revendications

1. Composition pharmaceutique comprenant un composé de formule : 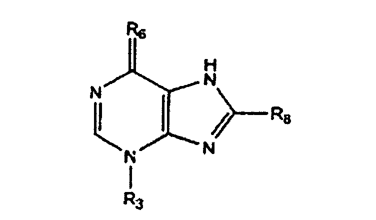 dans laquelle :
R₆ = S ou O
R₃ est choisi dans le groupe constitué des alkyles linéaires ou ramifiés en C₁-C₈; les alcènes linéaires ou ramifiés en C₂-C₈; les alcynes linéaires ou ramifiés en C₂-C₈ ; les cycloalkyles en C₃-₈; Q, et K ;
R₈ est choisi dans le groupe constitué des alkyles linéaires ou ramifiés en C₁-C₈ ; les alcènes linéaires ou ramifiés en C₂-C₈ ; les alcynes linéaires ou ramifiés en C₂-C₈ ; les cycloalkyles en C₃-₈; Q, et K ;
dans lesquels :
Q a la formule générale : 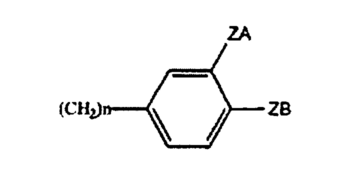 dans laquelle :
n = 0 ou 1 ;
Z = une liaison, CH₂, NH, O ou S ;
A et B peuvent former un cycle en ajoutant 1-3 groupes CH₂ lorsque Z=CH₂, NH, O ou S; et
A et B ne forment pas un cycle lorsque Z est une liaison, dans laquelle A et B sont indépendamment choisis dans le groupe comprenant hydrogène ; halogène ; alkyle en C₁-C₈ ; alkoxy en C₁-C₈ ; cycloalkoxy en C₃-C₈ ; hydroxy ; phényl ; benzyl ; et benzyloxy ; dans lesquels lesdits phényl, benzyl et benzyloxy sont éventuellement substitués par halogène, alkyle en C₁-C₈, alkoxy en C₁-C₈, cycloalkyl en C₃-C₈, cycloalkoxy en C₃-C₈ et hydroxy ;
K a la formule générale : 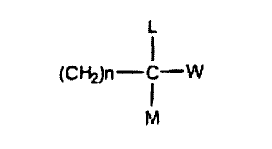 dans laquelle :
n=0 ou 1 ;
L et M sont indépendamment choisis dans le groupe comprenant hydrogène et méthyl ;
W est choisi dans le groupe constitué de Q ; hydroxy ; benzyloxy éventuellement substitué par halogène, alkyle en C₁-C₈, alkoxy en C₁-C₈, cycloalkyle en C₃-C₈, cycloalkoxy en C₃-C₈ et hydroxy ; aryle ; hétéroaryle ; et un cycle hétérocyclique ; à condition que, lorsque R₃ est un méthyl, R₈ n'est pas un hydrogène ; ainsi que les sels pharmaceutiquement acceptables ;
et au moins un excipient pharmaceutiquement acceptable ; ladite composition se présentant sous forme solide, ladite forme étant choisie dans le groupe constitué des comprimés, gelcap, caplet, granulé et pastille.

2. Composition pharmaceutique selon la revendication 1, **caractérisée en ce que** R₃ est un benzyle.

3. Composition pharmaceutique selon la revendication 1, **caractérisée en ce que** R₃ est un benzyle substitué avec un alkoxy et un groupe cycloalkoxy.

4. Composition pharmaceutique selon la revendication 1, **caractérisée en ce que** R₃ est un benzyle substitué avec un benzyloxy.

5. Composition pharmaceutique selon la revendication 1, choisie dans le groupe comprenant :
- 3-butyl-hypoxanthine ;
- 3-butyl-thiohypoxanthine ;
- 3-ethyl-hypoxanthine ;
- 3-ethyl-thiohypoxanthine ;
- 3,8-diethyl-hypoxanthine ;
- 3,8-diethyl-thiohypoxanthine ;
- 3-ethyl-8-cyclopropyl-hypoxanthine ;
- 3-ethyl-8-cyclopropyl-thiohypoxanthine ;
- 3-propyl-hypoxanthine ;
- 3-hexyl-hypoxanthine ;
- 3-hexyl-thiohypoxanthine ;
- 3-benzyl-hypoxanthine ;
- 3-benzyl-thiohypoxanthine ;
- 3-(4-methyl-butyl)-hypoxanthine ;
- 3-(4-methyl-butyl)-thiohypoxanthine ;
- 3-(2-methyl-butyl)-hypoxanthine ;
- 3-(2-methyl-butyl)-thiohypoxanthine ;
- 3-(3-cyclopentyloxy-4-methoxy-benzyl)-hypoxanthine ;
- 3-(3-cyclopentyloxy-4-methoxy-benzyl)-8(1-hydroxy-1-methyl-ethyl)-hypoxanthine ;
- 3-(3-cyclopentyloxy-4-methoxy-benzyl)-8(1-methyl-ethylene)-hypoxanthine ;
- 3-(3-cyclopentyloxy-4-methoxy-benzyl)-8(1-benzyloxy-1-methyl-ethyl)-hypoxanthine ;
- 3-(3-cyclopentyloxy-4-methoxy-benzyl)-8-benzyloxymethyl)-hypoxanthine ;
- 3-(3-cyclopentyloxy-4-methoxy-benzyl)-8-(1-methyl-ethyl)-hypoxanthine ;
- 3-(3-cyclopentyloxy-4-methoxy-benzyl)-8(1-(4-methoxybenzyloxy)-1-methyl-ethyl-hypoxanthine ;
- 3-(3-cyclopentyloxy-4-methoxy-benzyl)-8-(1-(4-fluorobenzyloxy)-1-methyl-ethyl)-hypoxanthine ;
- 3-(3-cyclopentyloxy-4-methoxy-benzyl)-8-(1-benzyloxy-1-methyl-ethyl)-hypoxanthine ;
- 3-(3-4-dimethoxy-benzyl)-8-(1-benzyloxy-1-methyl-ethyl)-hypoxanthine ;
- 3-(3-benzyloxy-4-methoxy-benzyl)-8-(1-methyl-ethyl)-hypoxanthine ;
- 3-(3-hydroxy-4-methoxy-benzyl)-8-(1-methyl-ethyl)-hypoxanthine ;
- 3-(3-4-dimethoxy-benzyl)-8-(1-methyl-ethyl)-hypoxanthine ;
- 3-(1,3-benzodioxole-5-methyl)-8(1-methyl-ethyl)-hypoxanthine ;
- 3-(4-chloro-benzyl)-8-(1-methyl-ethyl)-hypoxanthine ;
- 3-(3-chloro-benzyl)-8-(1-methyl-ethyl)-hypoxanthine ;
- 3-(4-methoxy-benzyl)-8-(1-(4-fluorobenzyloxy)-1-methyl-ethyl)-hypoxanthine ;
- ainsi que les sels phamaceutiquement acceptables.

6. Méthode de préparation d'une composition pharmaceutique conformément à l'une des revendications 1 à 5, pour le traitement d'un mammifère souffrant d'une maladie choisie dans le groupe comprenant l'asthme, les allergies, l'inflammation, la dépression, la démence et les états maladifs associés à des taux physiologiques anormalement élevés de cytokines.
